# EUROPEAN PATENT APPLICATION

(11) **EP 4 364 688 A1**
(43) Date of publication of application: **08.05.2024**
(21) Application number: 22833211.0
(22) Date of filing: 29.06.2022
(51) Int. Cl.: A61B 34/30

(54) **OPERATOR-SIDE DEVICE AND SURGERY ASSISTANCE SYSTEM**

(30) Priority: 30.06.2021 JP 2021109575; 15.11.2021 JP 2021185942
(71) Applicant: Kawasaki Jukogyo Kabushiki Kaisha, Hyogo 650-8670 (JP); Medicaroid Corporation, Hyogo 650-0047 (JP)
(72) Inventor: UEDA, Tatsuya, Kobe-shi, Hyogo 650-8670 (JP); MOMMA, Kazuya, Kobe-shi, Hyogo 650-8670 (JP); SHIBATA, Hiroki, Kobe-shi, Hyogo 650-8670 (JP); KUNO, Hirotaka, Kobe-shi, Hyogo 650-8670 (JP)
(74) Representative: Hoffmann Eitle
(86) International application number: PCT/JP2022/025972
(87) International publication number: WO 2023/277065

(57) **Abstract**

An operator-side apparatus (2) includes a first lever member (21bb) attached to a support member (21ba) so as to rotate with respect to the support member (21ba), and a first switch (21bf) attached to the first lever member (21bb) to perform a predetermined function.

## Description

### Technical Field

The present disclosure relates to an operator-side apparatus and a robotic surgical system, and more particularly, it relates to an operator-side apparatus and a robotic surgical system, each of which operates a patient-side apparatus including a patient-side arm having a tip end to which a surgical instrument is attached.

### Background Art

Conventionally, an operator-side apparatus that operates a patient-side apparatus including a patient-side arm having a tip end to which a surgical instrument is attached is known. Such an operator-side apparatus is disclosed in U.S. Patent No. 8,638,057, for example.

U.S. Patent No. 8,638,057 discloses a console that operates a robotic surgical manipulator including a robot arm having a tip end to which a surgical tool is attached. The console includes a master grip that is operated by the fingers of an operator such as a surgeon. The master grip is used to generate control signals to control the surgical tool. The master grip includes a tubular support structure, and a first grip and a second grip each having one end supported by the tubular support structure. A slidable switch that can be operated by the fingers of the operator is attached to the tubular support structure. The switch performs a clutch function.

### Prior Art

### Patent Document

Patent Document 1: U.S. Patent No. 8,638,057

### Summary of the Invention

However, in the console described in U.S. Patent No. 8,638,057, the switch is attached to the tubular support structure, and thus the operability of the switch operated by the hand of the operator may be poor.

The present disclosure is intended to solve the above problem. The present disclosure aims to provide an operator-side apparatus and a robotic surgical system each capable of improving the operability of a switch operated by the hand of an operator when the operator of an operator-side apparatus operates a patient-side apparatus including a patient-side arm having a tip end to which a surgical instrument is attached.

In order to attain the aforementioned object, an operator-side apparatus according to a first aspect of the present disclosure is operable to operate a patient-side apparatus including a patient-side arm having a tip end to which a surgical instrument is attached, and includes an operation arm, a support member connected to a proximal end of the operation arm, a first lever member attached to the support member so as to rotate with respect to the support member, and a first switch attached to the first lever member to perform a predetermined function.

As described above, the operator-side apparatus according to the first aspect of the present disclosure includes the first switch attached to the first lever member to perform the predetermined function. Accordingly, unlike a case in which the first switch is attached to the support member to perform the predetermined function, the first switch can be moved (rotated) by rotating the first lever, and thus the first switch can be moved to a position at which the first switch can be easily operated by an operator. Consequently, the first switch can be easily operated, and thus when the operator of the operator-side apparatus operates the patient-side apparatus including the patient-side arm having the tip end to which the surgical instrument is attached, the operability of the first switch operated by the hand of the operator can be improved.

A robotic surgical system according to a second aspect of the present disclosure includes a patient-side apparatus including a patient-side arm having a tip end to which a surgical instrument is attached, and an operator-side apparatus operable to operate the patient-side apparatus. The operator-side apparatus includes an operation arm, a support member connected to a proximal end of the operation arm, a lever member attached to the support member so as to rotate with respect to the support member, and a switch attached to the lever member to perform a predetermined function.

As described above, the robotic surgical system according to the second aspect of the present disclosure includes the switch attached to the lever member to perform the predetermined function. Accordingly, unlike a case in which the switch is attached to the support member to perform the predetermined function, the switch can be moved (rotated) by rotating the lever, and thus the switch can be moved to a position at which the switch can be easily operated by an operator. Consequently, the switch can be easily operated, and thus it is possible to provide the robotic surgical system capable of improving the operability of the switch operated by the hand of the operator when the operator of the operator-side apparatus operates the patient-side apparatus including the patient-side arm having the tip end to which the surgical instrument is attached.

An operator-side apparatus according to a third aspect of the present disclosure is operable to operate a patient-side apparatus including a patient-side arm having a tip end to which a surgical instrument is attached, and includes an operation arm, a support member connected to a proximal end of the operation arm, a lever member attached to the support member so as to rotate with respect to the support member, and a finger rest attached to the lever member and on which a finger of an operator is placed.

As described above, the operator-side apparatus according to the third aspect of the present disclosure includes the finger rest attached to the lever member and on which the finger of the operator is placed. Accordingly, the operator can rest his or her finger by placing it on the finger rest, and thus it is possible to provide the operator-side apparatus capable of mitigating fatigue on the finger of the operator.

A robotic surgical system according to a fourth aspect of the present disclosure includes a patient-side apparatus including a patient-side arm having a tip end to which a surgical instrument is attached, and an operator-side apparatus operable to operate the patient-side apparatus. The operator-side apparatus includes an operation arm, a support member connected to a proximal end of the operation arm, a lever member attached to the support member so as to rotate with respect to the support member, and a finger rest attached to the lever member and on which a finger of an operator is placed.

As described above, the robotic surgical system according to the fourth aspect of the present disclosure includes the finger rest attached to the lever member and on which the finger of the operator is placed. Accordingly, the operator can rest his or her finger by placing it on the finger rest, and thus it is possible to provide the robotic surgical system capable of mitigating fatigue on the finger of the operator.

According to the present disclosure, it is possible to improve the operability of the switch operated by the hand of the operator when the operator of the operator-side apparatus operates the patient-side apparatus including the patient-side arm having the tip end to which the surgical instrument is attached.

### Brief Description of the Drawings

FIG. 1 is a diagram showing the configuration of a surgical system according to a first embodiment.
FIG. 2 is a diagram showing the configuration of a patient-side apparatus according to the first embodiment.
FIG. 3 is a diagram showing the configuration of a robot arm of the patient-side apparatus according to the first embodiment.
FIG. 4 is a perspective view showing the configuration of an operation arm of a remote control apparatus according to the first embodiment.
FIG. 5 is a perspective view showing the configuration of the operation arm and an operation handle according to the first embodiment.
FIG. 6 is a perspective view (1) showing the configuration of the operation handle according to the first embodiment.
FIG. 7 is a perspective view (2) showing the configuration of the operation handle according to the first embodiment.
FIG. 8 is a perspective view showing a state in which an operator grasps the operation handle according to the first embodiment with his or her hand.
FIG. 9 is a plan view showing the operation handle according to the first embodiment.
FIG. 10 is a rear view showing the operation handle according to the first embodiment.
FIG. 11 is a side view showing the operation handle according to the first embodiment.
FIG. 12 is a diagram for illustrating inclination of a switch of the operation handle according to the first embodiment.
FIG. 13 is a sectional view showing the switch according to the first embodiment.
FIG. 14 is a perspective view for illustrating a flexible substrate of the operation handle according to the first embodiment.
FIG. 15 is a plan view for illustrating the flexible substrate of the operation handle according to the first embodiment.
FIG. 16 is a perspective view (1) showing the configuration of an operation handle according to a second embodiment.
FIG. 17 is a perspective view (2) showing the configuration of the operation handle according to the second embodiment.
FIG. 18 is a side view showing the configuration of the operation handle according to the second embodiment.
FIG. 19 is an exploded perspective view for illustrating the configuration of a switch according to the second embodiment.
FIG. 20 is a sectional view (1) for illustrating the configuration of the switch according to the second embodiment.
FIG. 21 is a sectional view (2) for illustrating the configuration of the switch according to the second embodiment.
FIG. 22 is a perspective view showing operation pedals according to the second embodiment.
FIG. 23 is a diagram for illustrating a setting screen according to the second embodiment.
FIG. 24 is a perspective view (1) showing the configuration of an operation handle according to a third embodiment.
FIG. 25 is a perspective view (2) showing the configuration of the operation handle according to the third embodiment.
FIG. 26 is a side view showing the configuration of the operation handle according to the third embodiment.
FIG. 27 is an exploded perspective view for illustrating the configuration of a switch according to the third embodiment.
FIG. 28 is a sectional view (1) for illustrating the configuration of the switch according to the third embodiment.
FIG. 29 is a sectional view (2) for illustrating the configuration of the switch according to the third embodiment.
FIG. 30 is a perspective view showing the configuration of an operation handle according to a first modified example.
FIG. 31 is a perspective view showing the configuration of an operation handle according to a second modified example.
FIG. 32 is a perspective view showing the configuration of an operation handle according to a third modified example.

### Modes for Carrying Out the Invention

A first embodiment embodying the present disclosure is hereinafter described on the basis of the drawings.

### First Embodiment

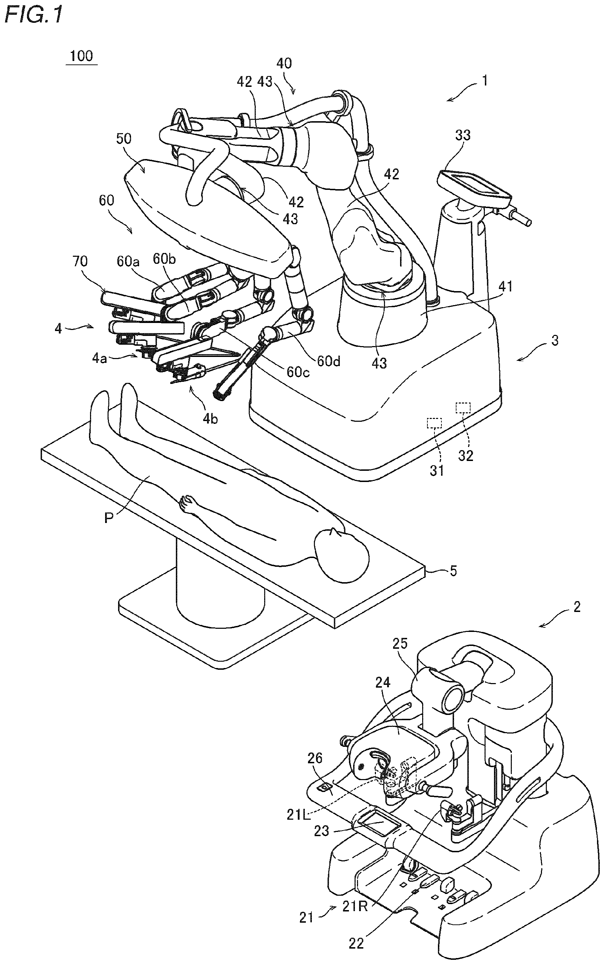

The configuration of a surgical system 100 according to the first embodiment is now described with reference to FIGS. 1 to 15.

As shown in FIG. 1, the surgical system 100 includes a medical manipulator 1 that is a patient-side apparatus, and a remote control apparatus 2 that is an operator-side apparatus to operate the medical manipulator 1. The medical manipulator 1 includes a medical cart 3 and is movable. The remote control apparatus 2 is spaced apart from the medical manipulator 1, and the medical manipulator 1 is remotely operated by the remote control apparatus 2. A surgeon inputs a command to the remote control apparatus 2 to cause the medical manipulator 1 to perform a desired operation. The remote control apparatus 2 transmits the input command to the medical manipulator 1. The medical manipulator 1 operates based on the received command. The medical manipulator 1 is arranged in an operating room that is a sterilized sterile field. The surgical system 100 is an example of a robotic surgical system. The medical manipulator 1 and the remote control apparatus 2 are examples of a patient-side apparatus and an operator-side apparatus, respectively.

The remote control apparatus 2 is arranged inside or outside the operating room, for example. The remote control apparatus 2 includes an operation manipulator 21, operation pedals 22, a touch panel 23, a monitor 24, a support arm 25, and a support bar 26. The operation manipulator 21 includes operation handles for the surgeon to input commands. The operation manipulator 21 receives the amount of operation for a surgical instrument 4. The monitor 24 is a scope-type display that displays an image captured by an endoscope 4b. The support arm 25 supports the monitor 24 so as to align the height of the monitor 24 with the height of the face of the surgeon. The touch panel 23 is arranged on the support bar 26. The head of the surgeon is detected by a sensor provided in the vicinity of the monitor 24 such that the medical manipulator 1 can be operated by the remote control apparatus 2. The surgeon operates the operation manipulator 21 and the operation pedals 22 while visually recognizing an affected area on the monitor 24. Thus, a command is input to the remote control apparatus 2. The command input to the remote control apparatus 2 is transmitted to the medical manipulator 1. The operation manipulator 21 includes a right-hand operation manipulator 21R and a left-hand operation manipulator 21L.

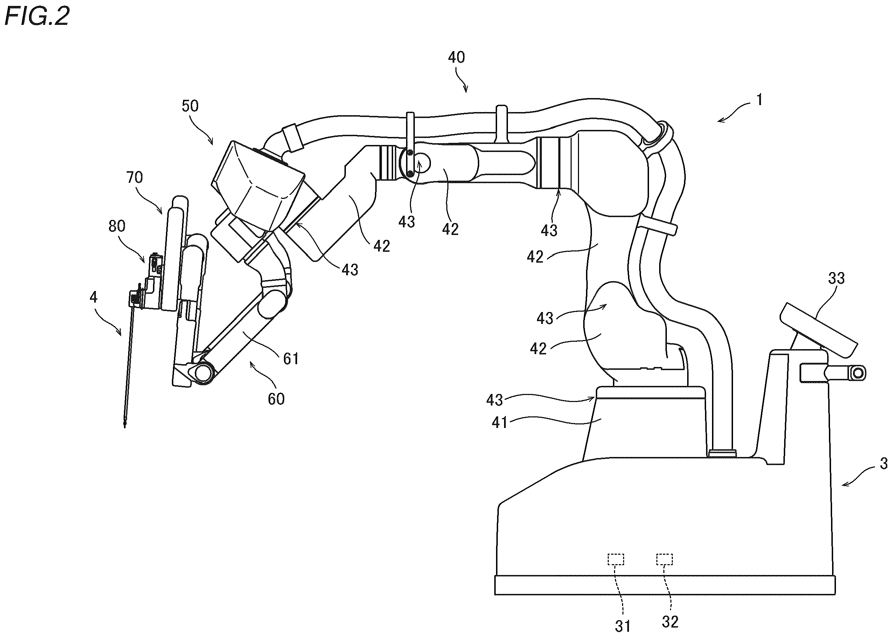

As shown in FIGS. 1 and 2, the medical cart 3 includes a controller 31 that controls the operation of the medical manipulator 1 and a storage 32 that stores programs or the like to control the operation of the medical manipulator 1. The controller 31 of the medical cart 3 controls the operation of the medical manipulator 1 based on the command input to the remote control apparatus 2.

The medical cart 3 includes an input 33. The input 33 receives operations to move a positioner 40, an arm base 50, and a plurality of manipulator arms 60 or change their postures mainly in order to prepare for surgery before the surgery.

The medical manipulator 1 shown in FIGS. 1 and 2 is arranged in the operating room. The medical manipulator 1 includes the medical cart 3, the positioner 40, the arm base 50, and the plurality of manipulator arms 60. The arm base 50 is attached to the tip end of the positioner 40. The arm base 50 has a relatively long rod shape. The bases of the plurality of manipulator arms 60 are attached to the arm base 50. Each of the plurality of manipulator arms 60 is able to take a folded and stored posture. The arm base 50 and the plurality of manipulator arms 60 are covered with sterile drapes and used.

The positioner 40 includes a 7-axis articulated robot, for example. The positioner 40 is arranged on the medical cart 3. The positioner 40 moves the arm base 50. Specifically, the positioner 40 moves the position of the arm base 50 three-dimensionally.

The positioner 40 includes a base 41 and a plurality of links 42 coupled to the base 41. The plurality of links 42 are coupled to each other by joints 43.

Surgical instruments 4 are attached to the tip ends of the plurality of manipulator arms 60. The surgical instruments 4 include a replaceable instrument 4a and the endoscope 4b, for example. The manipulator arms 60 are examples of a patient-side arm.

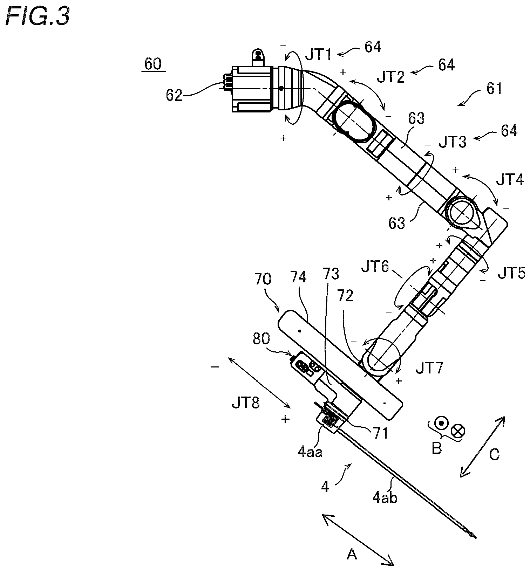

The configuration of the manipulator arms 60 is now described in detail with reference to FIG. 3.

As shown in FIG. 3, each of the manipulator arms 60 includes an arm portion 61 and a translation mechanism 70 provided at the tip end of the arm portion 61. The tip end side of the manipulator arm 60 three-dimensionally moves with respect to the arm base 50 on the base side of the manipulator arm 60. The manipulator arm 60 has eight degrees of freedom. Specifically, the manipulator arm 60 includes JT1 to JT7 axes as rotation axes and a JT8 axis as a linear motion axis. The plurality of manipulator arms 60 have the same or similar configurations as each other. The arm portion 61 includes a base 62, links 63, and joints 64.

The translation mechanism 70 is provided at the tip end of the arm portion 61, and the surgical instrument 4 is attached thereto. The translation mechanism 70 translates the surgical instrument 4 in a direction in which the surgical instrument 4 is inserted into a patient P placed on a surgical table 5. Furthermore, the translation mechanism 70 translates the surgical instrument 4 relative to the arm portion 61. Specifically, the translation mechanism 70 includes a holder 71 that holds the surgical instrument 4. Servomotors are housed in the holder 71. The servomotors rotate rotary bodies provided in a driven unit 4aa of the surgical instrument 4. The rotary bodies of the driven unit 4aa are rotated such that the surgical instrument 4 operates.

The arm portion 61 includes a 7-axis articulated robot arm. The arm portion 61 includes the base 62 to attach the arm portion 61 to the arm base 50, and a plurality of links 63 coupled to the base 62. The plurality of links 63 are coupled to each other by the joints 64.

The translation mechanism 70 translates the surgical instrument 4 attached to the holder 71 along an A direction in which a shaft 4ab extends by translating the holder 71 along the A direction. Specifically, the translation mechanism 70 includes a base end side link 72 connected to the tip end of the arm portion 61, a tip end side link 73, and a coupling link 74 provided between the base end side link 72 and the tip end side link 73. The holder 71 is provided on the tip end side link 73.

The coupling link 74 of the translation mechanism 70 is configured as a double speed mechanism that moves the tip end side link 73 relative to the base end side link 72 along the A direction. The tip end side link 73 is moved along the A direction relative to the base end side link 72 such that the surgical instrument 4 attached to the holder 71 is translated along the A direction. The tip end of the arm portion 61 is connected to the base end side link 72 so as to rotate the base end side link 72 about a B direction orthogonal to the A direction.

As shown in FIG. 1, the endoscope 4b is attached to one of the plurality of manipulator arms 60, such as a manipulator arm 60c, and instruments 4a that are surgical instruments 4 other than the endoscope 4b are attached to the remaining manipulator arms 60a, 60b, and 60d. A pivot position is taught with the endoscope 4b attached to the manipulator arm 60 to which the endoscope 4b is to be attached. Furthermore, pivot positions are taught with pivot position teaching instruments attached to the manipulator arms 60 to which the instruments 4a other than the endoscope 4b are to be attached. The endoscope 4b is attached to one of two manipulator arms 60b and 60c arranged in the center among four manipulator arms 60 arranged adjacent to each other. That is, the pivot position is individually set for each of the plurality of manipulator arms 60.

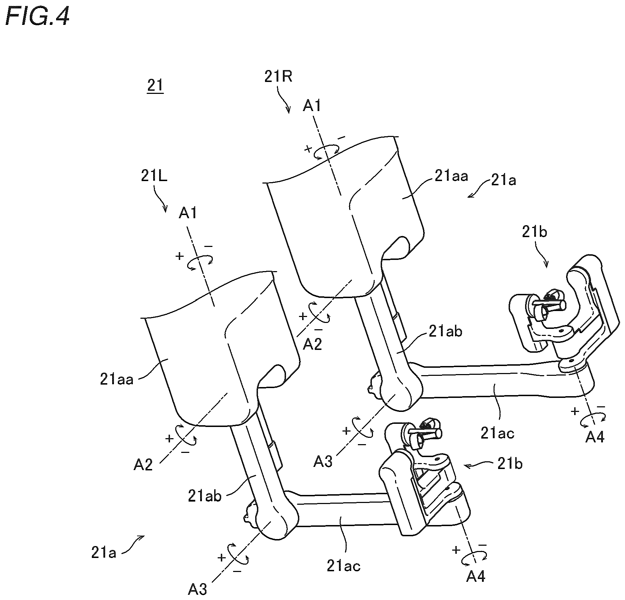

The configuration of the operation manipulator 21 is now described in detail with reference to FIGS. 4 to 15.

As shown in FIG. 4, the operation manipulator 21 includes the operation manipulator 21L located on the left side as viewed from an operator such as a surgeon and operated by the left hand of the operator, and the operation manipulator 21R located on the right side and operated by the right hand of the operator. The configurations of the operation manipulator 21L and the operation manipulator 21R are the same as or similar to each other except that operation handles 21b thereof have bilaterally symmetrical structures.

The operation manipulator 21 includes operation arms 21a and the operation handles 21b. Each of the operation arms 21a includes a link 21aa, a link 21ab, and a link 21ac. The upper end side of the link 21aa is attached to a main body of the remote control apparatus 2 such that the link 21aa is rotatable about an A1 axis along a vertical direction. The upper end side of the link 21ab is attached to the lower end side of the link 21aa such that the link 21ab is rotatable about an A2 axis along a horizontal direction. A first end side of the link 21ac is attached to the lower end side of the link 21ab such that the link 21ac is rotatable about an A3 axis along the horizontal direction.

The operation arms 21a support the operation handles 21b such that the operation handles 21b are movable within predetermined three-dimensional operation ranges. Specifically, the operation arms 21a support the operation handles 21b such that the operation handles 21b are movable in an upward-downward direction, a right-left direction, and a forward-rearward direction. The manipulator arms 60 are moved three-dimensionally so as to correspond to the three-dimensional operations of the operation arms 21a.

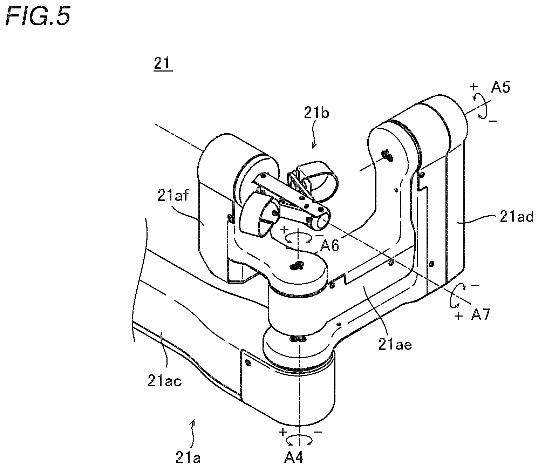

As shown in FIG. 5, the operation arm 21a of the right-hand operation manipulator 21R further includes a link 21ad, a link 21ae, and a link 21af. A first end side of the link 21ad is attached to a second end side of the link 21ac such that the link 21ad is rotatable about an A4 axis. A first end side of the link 21ae is attached to a second end side of the link 21ad such that the link 21ae is rotatable about an A5 axis. A first end side of the link 21af is attached to a second end side of the link 21ae such that the link 21af is rotatable about an A6 axis.

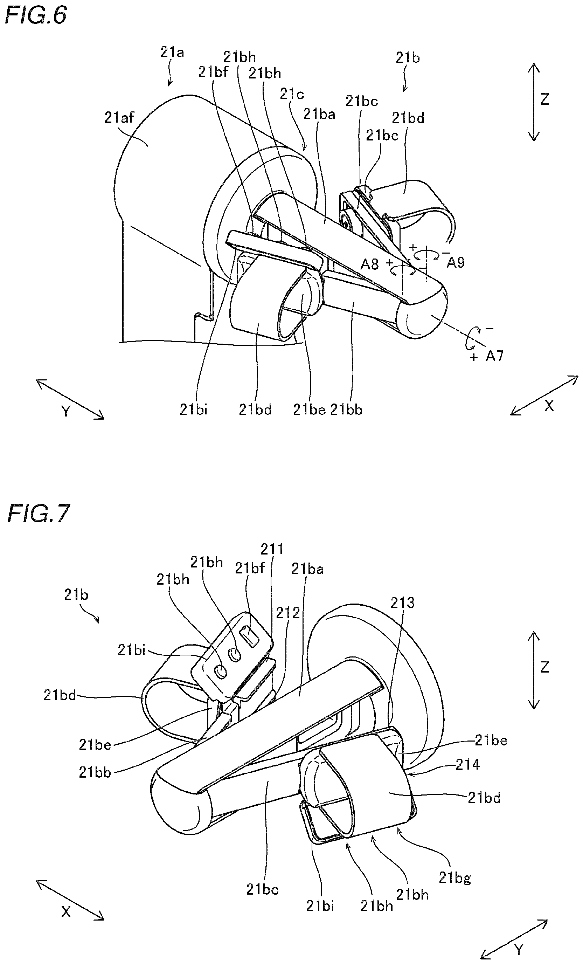

As shown in FIG. 6, the operation handle 21b of the right-hand operation manipulator 21R includes a support member 21ba, a first lever member 21bb, and a second lever member 21bc. The support member 21ba has a cylindrical shape extending in a Y direction, and is connected to the proximal end of the operation arm 21a. The distal end of the support member 21ba is connected to the proximal end of the operation arm 21a so as to rotate about an A7 axis parallel to the Y direction that is the longitudinal direction of the support member 21ba. That is, the distal end of the support member 21ba is connected to the link 21af so as to rotate about the A7 axis. The distal end of the support member 21ba and the proximal end of the operation arm 21a are connected to each other via a flange 21c. The A7 axis is an example of a first axis.

The first lever member 21bb has an elongated plate shape, and is attached to the support member 21ba so as to rotate with respect to the support member 21ba. The first lever member 21bb is attached to the support member 21ba so as to rotate about an A8 axis perpendicular to the A7 axis with respect to the support member 21ba. The first lever member 21bb faces the second lever member 21bc via the support member 21ba. The A8 axis is an example of a second axis.

The second lever member 21bc has an elongated plate shape, and is attached to the support member 21ba so as to rotate with respect to the support member 21ba. The second lever member 21bc is attached to the support member 21ba so as to rotate about an A9 axis perpendicular to the A7 axis with respect to the support member 21ba. The second lever member 21bc faces the first lever member 21bb via the support member 21ba. The A9 axis is an example of a third axis.

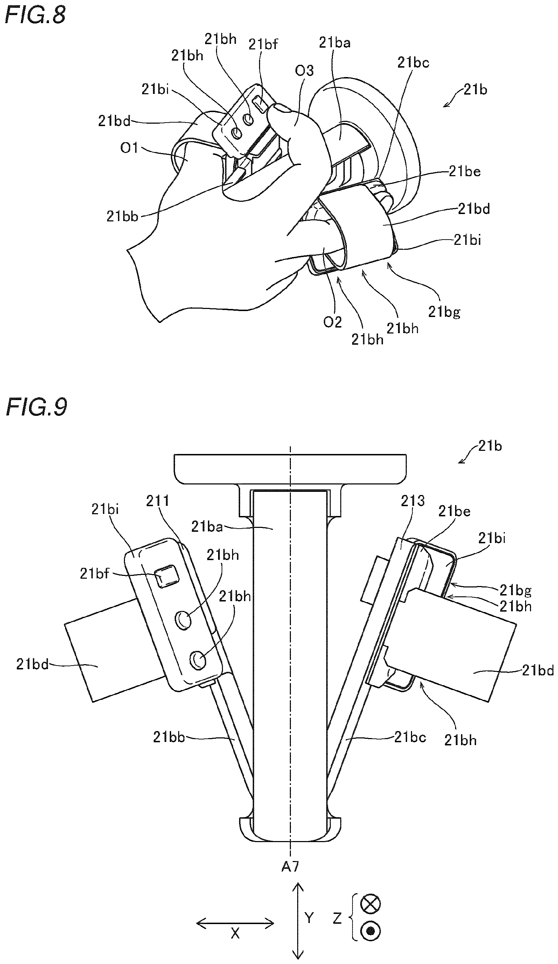

A cylindrical finger insertion portion 21bd and a rectangular finger pad 21be are arranged on each of the first lever member 21bb and the second lever member 21bc. The operator inserts his or her finger into the finger insertion portion 21bd, places the finger on the finger pad 21be, and operates the operation handle 21b. For example, the thumb O1 of the operator is inserted into the finger insertion portion 21bd on the first lever member 21bb side, and the middle finger O2 of the operator is inserted into the finger insertion portion 21bd on the second lever member 21bc side (FIG. 8).

In the first embodiment, as shown in FIG. 7, the operation handle 21b of the right-hand operation manipulator 21R further includes a first switch 21bf attached to the first lever member 21bb to perform a predetermined function. The first switch 21bf is a clutch switch that performs a clutch function of preventing an operation of the operator from being transmitted to the medical manipulator 1. As shown in FIG. 8, the operator operates the first switch 21bf with the index finger O3 of the right hand that is not operating the first lever member 21bb or the second lever member 21bc, for example. Furthermore, the operator operates the first switch 21bf by holding the first switch 21bf between the thumb O1 of the right hand that operates the first lever member 21bb and the index finger O3 of the right hand that operates the first switch 21bf, for example.

In the first embodiment, as shown in FIGS. 7, 9, and 11, the first switch 21bf is arranged closer to the distal end than the proximal end of the first lever member 21bb. Specifically, the first switch 21bf is arranged at the position of the distal end of the first lever member 21bb. Furthermore, the first switch 21bf is arranged at a first end 211 of the first end 211 and the second end 212 of the first lever member 21bb in the Z direction parallel to the A8 axis. That is, the first switch 21bf is arranged at the upper first end 211 with respect to the thumb O1 of the operator placed on the first lever member 21bb. In the first embodiment, the first end 211 is a portion of the distal end of the first lever member 21bb on a first side in the Z direction parallel to the A8 axis. The second end 212 is a portion of the distal end of the first lever member 21bb on a second side opposite to the first end 211 in the Z direction parallel to the A8 axis.

In the first embodiment, as shown in FIGS. 7, 9, 10, and 11, the operation handle 21b further includes a second switch 21bg attached to the second lever member 21bc to perform a predetermined function. The second switch 21bg has the same function as the first switch 21bf. That is, the second switch 21bg is a clutch switch that performs a clutch function of preventing an operation of the operator from being transmitted to the medical manipulator 1. The second switch 21bg is located at the same position as the first switch 21bf when the support member 21ba is rotated 180 degrees about the A7 axis. That is, the second switch 21bg can be operated in the same manner as the first switch 21bf.

In the first embodiment, as shown in FIGS. 7, 9, and 11, the second switch 21bg is arranged closer to the distal end than the proximal end of the second lever member 21bc. Specifically, the second switch 21bg is arranged at the position of the distal end of the second lever member 21bc. Furthermore, the second switch 21bg is arranged at a second end 214 of a first end 213 and the second end 214 of the second lever member 21bc in the Z direction parallel to the A9 axis. That is, the second switch 21bg is arranged at the second end 214 opposite to the first switch 21bf. In the first embodiment, the first end 213 is a portion of the distal end of the second lever member 21bc on the first side in the Z direction parallel to the A9 axis. The second end 214 is a portion of the distal end of the second lever member 21bc on the second side opposite to the first end 213 in the Z direction parallel to the A9 axis.

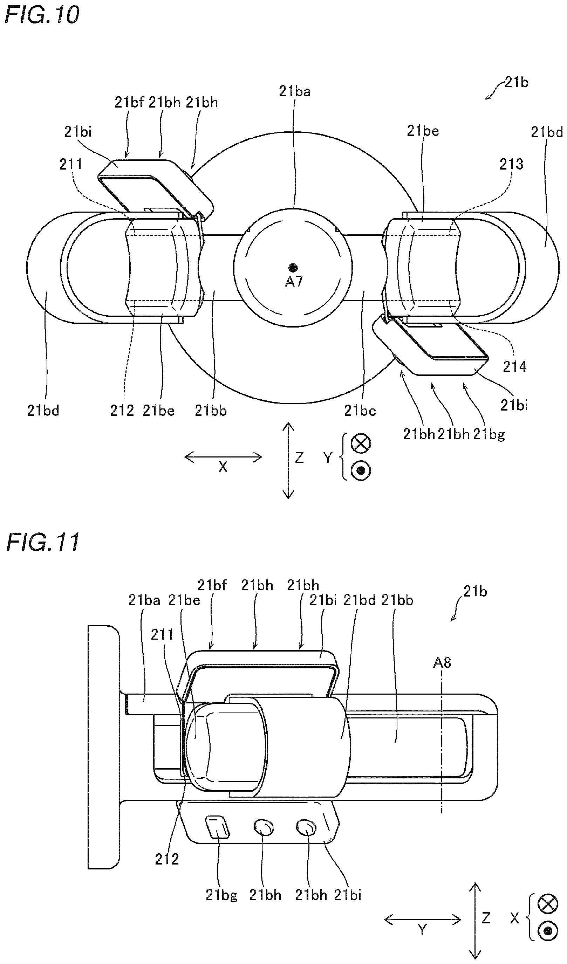

In the first embodiment, as shown in FIG. 9, the first switch 21bf and the second switch 21bg are arranged symmetrically about the A7 axis of the support member 21ba. Specifically, the first switch 21bf and the second switch 21bg are arranged line-symmetrically about the A7 axis of the support member 21ba as viewed in the Z direction parallel to the A8 axis and the A9 axis. More specifically, as shown in FIG. 10, the first switch 21bf and the second switch 21bg are arranged point-symmetrically about the rotation center of the A7 axis of the support member 21ba as viewed in the Y direction parallel to the A7 axis.

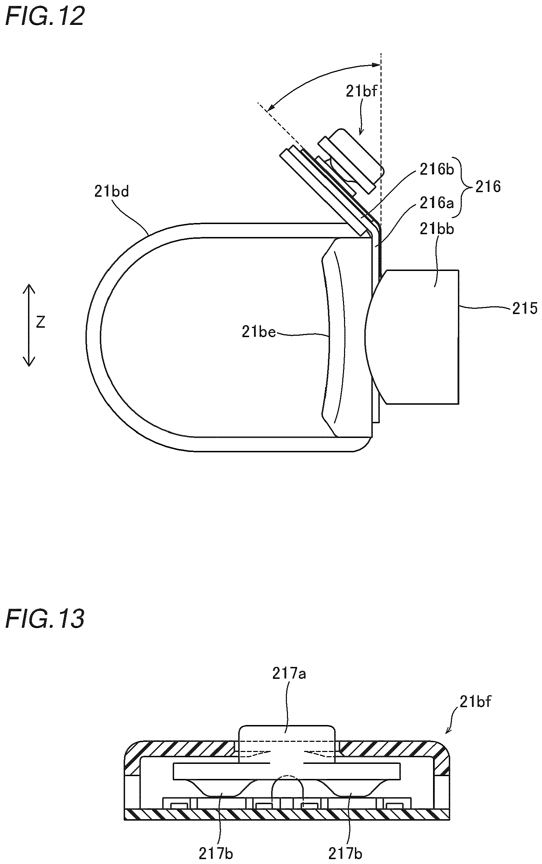

In the first embodiment, as shown in FIG. 12, the first switch 21bf is inclined with respect to an opposing surface 215 of the first lever member 21bb facing the support member 21ba. The first switch 21bf is inclined by a predetermined angle θ with respect to a plane parallel to the opposing surface 215 in a direction away from the support member 21ba.

In the first embodiment, the first lever member 21bb includes a plate-shaped attachment member 216 attached to the opposing surface 215 to support the first switch 21bf such that the first switch 21bf is inclined with respect to the opposing surface 215. The attachment member 216 includes a first flat plate 216a parallel to the opposing surface 215, and a second flat plate 216b connected to the first flat plate 216a and inclined so as to be bent with respect to the first flat plate 216a. The first switch 21bf is attached to the second flat plate 216b. The second flat plate 216b is inclined by the predetermined angle θ with respect to the first flat plate 216a in the direction away from the support member 21ba. Although the inclined configuration of the first switch 21bf has been described, the second switch 21bg has the same or similar inclined configuration.

In the first embodiment, as shown in FIG. 13, the first switch 21bf and the second switch 21bg are push-button. Specifically, the push-button first switch 21bf and the push-button second switch 21bg are clutch switches that perform a clutch function of preventing an operation of the operator from being transmitted to the medical manipulator 1, and are redundant switches. The first switch 21bf and the second switch 21bg as redundant switches each include one push-down operation unit 217a and two contact portions 217b. When the push-down operation unit 217a is depressed, the two contact portions 217b become conductive. Then, the clutch function is performed. When conduction of at least one of the two contact portions 217b is not detected, the remote control apparatus 2 detects a failure. In FIG. 13, only the first switch 21bf is shown for convenience.

In the first embodiment, as shown in FIGS. 7 to 11, the operation handle 21b further includes a plurality of customized switches 21bh to perform functions other than the clutch function of preventing an operation of the operator from being transmitted to the medical manipulator 1. Functions to be performed by the plurality of customized switches 21bh are customized by function assignment to the plurality of customized switches 21bh. Functions related to the surgery of the patient P, such as incision, suction, and drainage are assigned to the plurality of customized switches 21bh. The incision function refers to a function for incising a surgical site of the patient P using a high-frequency current with the instrument 4a. The suction function refers to a function for suctioning, with the instrument 4a, liquid such as washing water used to wash the surgical site of the patient P or blood generated at the surgical site of the patient P. The drainage function refers to a function for supplying, through the instrument 4a, washing water to the surgical site of the patient P to clean the surgical site of the patient P. The operator assigns a desired function to the customized switch 21bh at a desired position by operating the remote control apparatus 2 before the surgery of the patient P.

In the first embodiment, a plurality of (two) customized switches 21bh are arranged on the first lever member 21bb. Specifically, the plurality of customized switches 21bh are arranged on the first lever member 21bb together with the first switch 21bf. The plurality of customized switches 21bh and the first switch 21bf are arranged in one switch housing 21bi such that operation surfaces thereof protrude to the outside and contacts thereof are housed inside. The plurality of customized switches 21bh are inclined with respect to the opposing surface 215, similarly to the first switch 21bf. The plurality of customized switches 21bh are push-button, similarly to the first switch 21bf. The operation surfaces of the plurality of customized switches 21bh and the first switch 21bf have different shapes from each other. The plurality of customized switches 21bh are arranged more proximally than the first switch 21bf. Among the plurality of customized switches 21bh and first switches 21bf, the first switch 21bf is arranged most distally. A plurality of (two) customized switches 21bh are also arranged on the second lever member 21bc. The configuration of the plurality of customized switches 21bh of the second lever member 21bc is the same as or similar to the configuration of the plurality of customized switches 21bh of the first lever member 21bb.

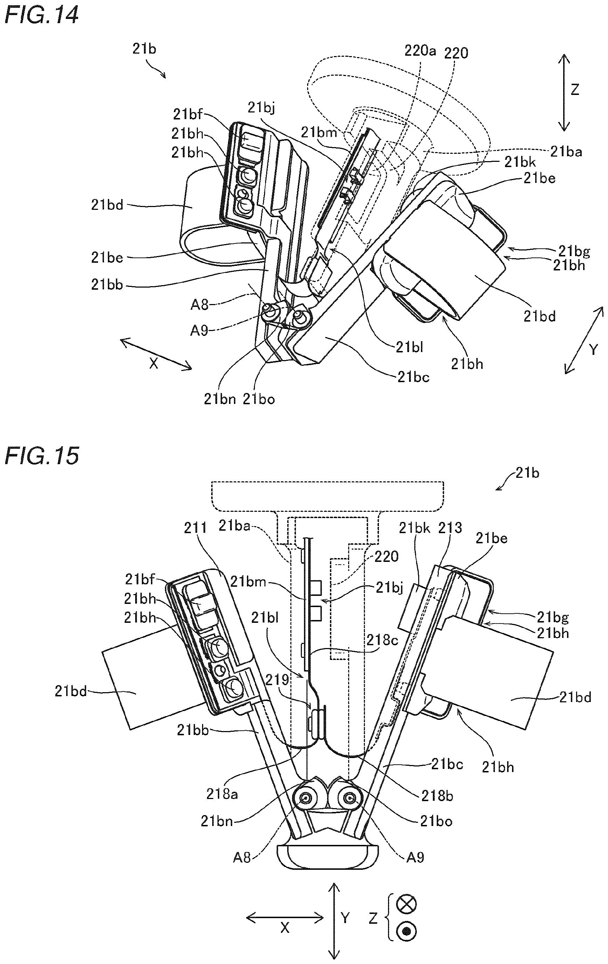

As shown in FIG. 14, the operation handle 21b further includes a sensor 21bj that detects the angles of the first lever member 21bb and the second lever member 21bc with respect to the support member 21ba. The sensor 21bj is a Hall sensor arranged on the support member 21ba to detect the magnetic field of a magnet 21bk arranged on the second lever member 21bc. The magnet 21bk may be placed on the first lever member 21bb. When the first lever member 21bb and the second lever member 21bc rotate with respect to the support member 21ba, a distance between the sensor 21bj and the magnet 21bk changes, and the magnitude of the magnetic field detected by the sensor 21bj changes. Thus, the sensor 21bj detects the angles of the first lever member 21bb and the second lever member 21bc with respect to the support member 21ba. The sensor 21bj is an example of an angle sensor.

In the first embodiment, the operation handle 21b includes a flexible printed circuit (FPC) 21bl in which the sensor 21bj, the first switch 21bf, and the second switch 21bg are arranged, and a reinforcing plate 21bm arranged at the position of the sensor 21bj of the FPC 21bl.

As shown in FIG. 15, the FPC 21bl is arranged from the support member 21ba to the first lever member 21bb or the second lever member 21bc. The FPC 21bl is branched into a first portion 218a in which the first switch 21bf is arranged and a second portion 218b in which the second switch 21bg is arranged. The first portion 218a is folded back in a U-shape from the proximal side of the support member 21ba toward the distal side of the first lever member 21bb. The second portion 218b is folded back in a U-shape from the proximal side of the support member 21ba toward the distal side of the first lever member 21bb. The FPC 21bl includes a third portion 218c that is a portion before branching into the first portion 218a and the second portion 218b. The sensor 21bj is arranged in the third portion 218c. The third portion 218c is reinforced at the position of the sensor 21bj by the reinforcing plate 21bm so as not to bend. The third portion 218c is arranged from the distal side to the proximal side of the support member 21ba.

An attachment member 219 to which the FPC 21bl is attached is arranged at a branch point between the first portion 218a and the second portion 218b of the FPC 21bl. The FPC 21bl is attached to the attachment member 219 in a folded manner so as to be branched into the first portion 218a and the second portion 218b. The attachment member 219 is arranged closer to the center of the support member 21ba in an X direction than the reinforcing plate 21bm. Thus, the branch point between the first portion 218a and the second portion 218b of the FPC 21bl can be arranged close to the center of the support member 21ba. Consequently, excessive bending of the U-shaped portion of the first portion 218a and the U-shaped portion of the second portion 218b can be reduced or prevented.

In the first portion 218a, the plurality of customized switches 21bh are arranged together with the first switch 21bf. That is, two types of switches, the first switch 21bf and the plurality of customized switches 21bh, are arranged in the first portion 218a. In the second portion 218b, the plurality of customized switches 21bh are arranged together with the second switch 21bg. That is, two types of switches, the second switch 21bg and the plurality of customized switches 21bh, are arranged in the second portion 218b.

As shown in FIGS. 14 and 15, the support member 21ba includes a recess 220 in which the magnet 21bk that protrudes from the second lever member 21bc is arranged when the second lever member 21bc is closed. The recess 220 faces the sensor 21bj. The recess 220 is recessed toward the sensor 21bj. The recess 220 allows the second lever member 21bc, on which the magnet 21bk is arranged, to be completely closed with respect to the support member 21ba. The recess 220 includes an opening 220a at a position facing the sensor 21bj. The opening 220a is a through-hole. The sensor 21bj is exposed with respect to the magnet 21bk via the opening 220a. The magnet 21bk protrudes toward the sensor 21bj on the second lever member 21bc.

The operation handle 21b further includes a first gear 21bn and a second gear 21bo to interlockingly rotate the first lever member 21bb and the second lever member 21bc. The first lever member 21bb and the second lever member 21bc are interlockingly opened and closed with respect to the support member 21ba by the first gear 21bn and the second gear bo. The first gear 21bn is connected to the proximal end of the first lever member 21bb so as to rotate together with the first lever member 21bb about the A8 axis. The second gear 21bo is connected to the proximal end of the second lever member 21bc so as to rotate together with the second lever member 21bc about the A9 axis. The teeth of the first gear 21bn and the second gear 21bo mesh with each other. The first gear 21bn and the second gear 21bo rotate in opposite directions when the first lever member 21bb and the second lever member 21bc rotate.

The first switch 21bf and the plurality of customized switches 21bh provided on the operation handle 21b of the right-hand operation manipulator 21R have been described above, but the operation handle 21b of the left-hand operation manipulator 21L has the same or similar structure as that of the operation handle 21b of the right-hand operation manipulator 21R except that the operation handle 21b of the left-hand operation manipulator 21L has a structure bilaterally symmetrical to that of the operation handle 21b of the right-hand operation manipulator 21R. That is, in the operation handle 21b of the left-hand operation manipulator 21L, the first switch 21bf and the plurality of customized switches 21bh are attached to the second lever member 21bc. The operator operates the first switch 21bf and the plurality of customized switches 21bh by holding the first switch 21bf and the plurality of customized switches 21bh between the thumb of the left hand that operates the second lever member 21bc and the index finger of the left hand that operates the first switch 21bf.

### Advantages of First Embodiment

According to the first embodiment, the following advantages are achieved.

According to the first embodiment, as described above, the remote control apparatus 2 includes the first switch 21bf attached to the first lever member 21bb to perform the predetermined function. Accordingly, unlike a case in which the first switch 21bf is attached to the support member 21ba to perform the predetermined function, the first switch can be moved (rotated) by rotating the first lever, and thus the first switch can be moved to a position at which the first switch can be easily operated by the operator. Consequently, the first switch 21bf can be easily operated, and thus when the operator of the remote control apparatus 2 operates the medical manipulator 1 including the manipulator arms 60 having tip ends to which the surgical instruments 4 are attached, the operability of the first switch 21bf operated by the hand of the operator can be improved. Furthermore, the first switch 21bf can be operated by being held between the finger of the operator that operates the first lever member 21bb and the finger of the operator that operates the first switch 21bf. Consequently, the first switch 21bf can be easily operated, and thus when the operator of the remote control apparatus 2 operates the medical manipulator 1 including the manipulator arms 60 having tip ends to which the surgical instruments 4 are attached, the operability of the first switch 21bf operated by the hand of the operator can be improved.

According to the first embodiment, as described above, the distal end of the support member 21ba is connected to the proximal end of the operation arm 21a so as to rotate about the A7 axis parallel to the longitudinal direction of the support member 21ba. The proximal end of the first lever member 21bb is attached to the support member 21ba so as to rotate about the A8 axis perpendicular to the A7 axis with respect to the support member 21ba. Accordingly, the operator can easily operate the support member 21ba such that the support member 21ba rotates about the A7 axis and can easily operate the first lever member 21bb such that the first lever member 21bb rotates about the A8 axis with respect to the support member 21ba, and the operability of the first switch 21bf operated by the hand of the operator can be improved.

According to the first embodiment, as described above, the first switch 21bf is arranged closer to the distal end than the proximal end of the first lever member 21bb. Accordingly, the first switch 21bf can be operated by being held between a portion closer to the tip end than the base end of the finger of the operator that operates the first lever member 21bb and the finger of the operator that operates the first switch 21bf. Consequently, the operability of the first switch 21bf operated by the hand of the operator can be further improved.

According to the first embodiment, as described above, the first switch 21bf is arranged at the first end 211 of the first end 211 and the second end 212 of the first lever member 21bb in the direction parallel to the A8 axis. Accordingly, the first switch 21bf can be arranged at a position at which the first switch 21bf can be easily held between the finger of the operator that operates the first lever member 21bb and the finger of the operator that operates the first switch 21bf, and thus the first switch 21bf can be easily operated.

According to the first embodiment, as described above, the remote control apparatus 2 includes the second lever member 21bc facing the first lever member 21bb via the support member 21ba, and the second switch 21bg attached to the second lever member 21bc to perform the predetermined function. The distal end of the support member 21ba is connected to the proximal end of the operation arm 21a so as to rotate about the A7 axis parallel to the longitudinal direction of the support member 21ba. The proximal end of the second lever member 21bc is attached to the support member 21ba so as to rotate about the A9 axis perpendicular to the A7 axis with respect to the support member 21ba. Accordingly, the second switch 21bg is attached to the second lever member 21bc such that the second switch 21bg can be operated in the same manner as the first switch 21bf when the support member 21ba rotates about the A7 axis. That is, the second switch 21bg can be operated by being held between the finger of the operator that operates the second lever member 21bc and the finger of the operator that operates the second switch 21bg. Consequently, the second switch 21bg can be easily operated, and thus the operability of the second switch 21bg operated by the hand of the operator can be improved. Furthermore, the operator can easily operate the support member 21ba such that the support member 21ba rotates about the A7 axis, and can easily operate the second lever member 21bc such that the second lever member 21bc rotates about the A9 axis with respect to the support member 21ba.

According to the first embodiment, as described above, the second switch 21bg is arranged closer to the distal end than the proximal end of the second lever member 21bc. Accordingly, the second switch 21bg can be operated by being held between a portion closer to the tip end than the base end of the finger of the operator that operates the second lever member 21bc and the finger of the operator that operates the second switch 21bg when the support member 21ba rotates about the A7 axis. Consequently, the operability of the second switch 21bg operated by the hand of the operator can be further improved.

According to the first embodiment, as described above, the second switch 21bg is arranged at the second end 214 of the first end 213 and second end 214 of the second lever member 21bc in the direction parallel to the A9 axis. Accordingly, the second switch 21bg can be arranged at a position at which the second switch 21bg can be easily held between the finger of the operator that operates the second lever member 21bc and the finger of the operator that operates the second switch 21bg when the support member 21ba rotates about the A7 axis, and thus the second switch 21bg can be easily operated.

According to the first embodiment, as described above, the first switch 21bf includes the clutch switch to perform the clutch function of preventing an operation of the operator from being transmitted to the medical manipulator 1. Accordingly, when the operator wants to perform the clutch function, the clutch function can be easily performed by the first switch 21bf that is operated by the hand of the operator and has improved operability.

According to the first embodiment, as described above, the first switch 21bf and the second switch 21bg are arranged symmetrically about the A7 axis of the support member 21ba. Accordingly, the first switch 21bf and the second switch 21bg are arranged symmetrically about the A7 axis of the support member 21ba, and thus the first switch 21bf and the second switch 21bg can be arranged in a balanced manner. When the first switch 21bf and the second switch 21bg are arranged point-symmetrically about the rotation center of the A7 axis of the support member 21ba, the first switch 21bf can be located at the same position as the second switch 21bg, and vice versa when the support member 21ba is rotated 180 degrees about the A7 axis. Consequently, when the support member 21ba is rotated 180 degrees about the A7 axis, the first switch 21bf can be operated at the same position as the second switch 21bg, and vice versa.

According to the first embodiment, as described above, the first switch 21bf is inclined with respect to the opposing surface 215 of the first lever member 21bb facing the support member 21ba. Accordingly, the first switch 21bf can be easily operated as compared with a case in which the first switch 21bf is arranged parallel to the opposing surface 215 of the first lever member 21bb facing the support member 21ba. Thus, the operability of the first switch 21bf can be improved.

According to the first embodiment, as described above, the first lever member 21bb includes the plate-shaped attachment member 216 attached to the opposing surface 215 to support the first switch 21bf such that the first switch 21bf is inclined with respect to the opposing surface 215. The attachment member 216 includes the first flat plate 216a parallel to the opposing surface 215, and the second flat plate 216b connected to the first flat plate 216a and inclined so as to be bent with respect to the first flat plate 216a. The first switch 21bf is attached to the second flat plate 216b. Accordingly, with a relatively simple structure, the first switch 21bf can be arranged so as to be inclined with respect to the opposing surface 215. Consequently, the operability of the first switch 21bf can be improved with a relatively simple structure.

According to the first embodiment, as described above, the first switch 21bf is push-button. Accordingly, unlike a slide switch, the predetermined function can be performed by the first switch 21bf by simply pressing the first switch 21bf, and thus when the operator wants to perform the predetermined function, the predetermined function can be easily performed by the push-button first switch 21bf.

According to the first embodiment, as described above, the push-button first switch 21bf is a clutch switch operable to perform the clutch function of preventing an operation of the operator from being transmitted to the medical manipulator 1, and is a redundant switch. Accordingly, the first switch 21bf serving as a clutch switch can be a redundant switch, and thus when the first switch 21bf serving as a clutch switch fails, the failure of the first switch 21bf serving as a clutch switch can be easily detected.

According to the first embodiment, as described above, the remote control apparatus 2 includes the plurality of customized switches 21bh to perform the functions other than the clutch function of preventing an operation of the operator from being transmitted to the medical manipulator 1. The functions to be performed by the plurality of customized switches 21bh are customized by function assignment to the plurality of customized switches 21bh. Accordingly, the operator can assign desired functions related to the surgery of the patient P, such as incision, suction, and drainage to the plurality of customized switches 21bh in a desired order. Consequently, the operator can smoothly perform surgery on the patient P using the plurality of customized switches 21bh to which desired functions have been assigned in a desired order.

According to the first embodiment, as described above, the plurality of customized switches 21bh are arranged on the first lever member 21bb. Accordingly, the functions having been assigned to the plurality of customized switches 21bh can be performed by operations by the hand of the operator. Consequently, the operator can easily perform the functions having been assigned to the plurality of customized switches 21bh.

According to the first embodiment, as described above, the remote control apparatus 2 includes the sensor 21bj to detect the angle of the first lever member 21bb with respect to the support member 21ba, the FPC 21bl in which the sensor 21bj and the first switch 21bf are arranged, and the reinforcing plate 21bm arranged at the position of the sensor 21bj of the FPC 21bl. Accordingly, the FPC 21bl can be used in common for the sensor 21bj and the first switch 21bf. Furthermore, the position of the sensor 21bj of the FPC 21bl can be supported by the reinforcing plate 21bm, and thus the sensor 21bj can be stably arranged.

### Second Embodiment

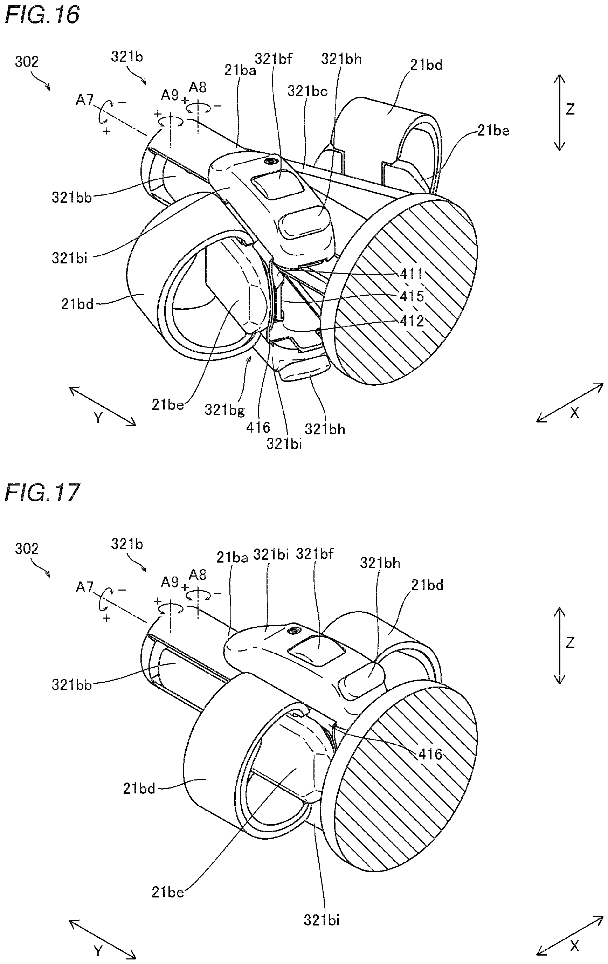

A remote control apparatus 302 according to a second embodiment is now described with reference to FIGS. 16 to 23. The same or similar configurations as those of the first embodiment are denoted by the same reference numerals, and detailed description thereof is omitted. The remote control apparatus 302 is an example of an operator-side apparatus.

As shown in FIGS. 16 and 17, the remote control apparatus 302 according to the second embodiment includes operation handles 321b in place of the operation handles 21b according to the first embodiment. The operation handle 321b of a right-hand operation manipulator 21R is described below. The configurations of a left-hand operation manipulator 21L and the right-hand operation manipulator 21R are the same as or similar to each other except that the operation handles 321b thereof have bilaterally symmetrical structures.

The operation handle 321b includes a support member 21ba, a first lever member 321bb, and a second lever member 321bc.

The first lever member 321bb has an elongated plate shape, and is attached to the support member 21ba so as to rotate with respect to the support member 21ba. The first lever member 321bb is attached to the support member 21ba so as to rotate about an A9 axis perpendicular to an A7 axis with respect to the support member 21ba. The first lever member 321bb faces the second lever member 321bc via the support member 21ba. The A9 axis is an example of a second axis.

The second lever member 321bc has an elongated plate shape, and is attached to the support member 21ba so as to rotate with respect to the support member 21ba. The second lever member 321bc is attached to the support member 21ba so as to rotate about an A8 axis perpendicular to the A7 axis with respect to the support member 21ba. The second lever member 321bc faces the first lever member 321bb via the support member 21ba. The A8 axis is an example of a third axis.

A finger insertion portion 21bd and a finger pad 21be are arranged on each of the first lever member 321bb and the second lever member 321bc.

In the second embodiment, the operation handle 321b further includes a first switch 321bf attached to the first lever member 321bb to perform a predetermined function. The first switch 321bf is a clutch switch that performs a clutch function of preventing an operation of an operator from being transmitted to a medical manipulator 1. For example, when the operator operates the first lever member 321bb with the middle finger of the right hand and operates the second lever member 321bc with the thumb of the right hand, the operator closes the first lever member 321bb and the second lever member 321bc (a state shown in FIG. 17) and operates the first switch 321bf with the index finger of the right hand that is not operating the first lever member 321bb or the second lever member 321bc.

In the second embodiment, as shown in FIGS. 16 to 18, the first switch 321bf is arranged closer to the distal end than the proximal end of the first lever member 321bb. Specifically, the first switch 321bf is arranged at the position of the distal end of the first lever member 321bb. Furthermore, the first switch 321bf is arranged at a first end 411 of the first end 411 and a second end 412 of the first lever member 321bb in a Z direction parallel to the A9 axis. The first end 411 is a portion of the distal end of the first lever member 321bb on a first side in the Z direction parallel to the A9 axis. The second end 412 is a portion of the distal end of the first lever member 321bb on a second side opposite to the first end 411 in the Z direction parallel to the A9 axis.

In the second embodiment, the operation handle 321b further includes a second switch 321bg attached to the first lever member 321bb to perform a predetermined function. In the second embodiment, the first switch 321bf and the second switch 321bg are arranged on the first lever member 321bb. The second switch 321bg has the same function as that of the first switch 321bf. That is, the second switch 321bg is a clutch switch that performs a clutch function of preventing an operation of the operator from being transmitted to the medical manipulator 1. For example, when the operator operates the first lever member 321bb with the index finger of the right hand and operates the second lever member 321bc with the thumb of the right hand, the operator closes the first lever member 321bb and the second lever member 321bc with respect to the support member 21ba (the state shown in FIG. 17), and operates the second switch 321bg with the middle finger of the right hand that is not operating the first lever member 321bb or the second lever member 321bc.

In the second embodiment, the second switch 321bg is arranged closer to the distal end than the proximal end of the first lever member 321bb. Specifically, the second switch 321bg is arranged at the position of the distal end of the first lever member 321bb. Furthermore, the second switch 321bg is arranged at the second end 412 of the first end 411 and the second end 412 of the first lever member 321bb in the Z direction parallel to the A8 axis. That is, the second switch 321bg is arranged at the second end 412 opposite to the first switch 321bf.

The first switch 321bf and the second switch 321bg are arranged symmetrically with the first lever member 321bb interposed between the first switch 321bf and the second switch 321bg in the Z direction parallel to the A8 axis and the A9 axis. Thus, when the operator prefers to operate the first lever member 321bb with the middle finger of the right hand, the operator can operate the first switch 321bf with the index finger of the right hand that is not operating the first lever member 321bb, and when the operator prefers to operate the first lever member 321bb with the index finger, the operator can operate the first switch 321bf with the middle finger of the right hand that is not operating the first lever member 321bb. Consequently, it is possible to accommodate both an operator who operates the first lever member 321bb with the middle finger of the right hand and an operator who operates the first lever member 321bb with the index finger of the right hand.

The first switch 321bf and the second switch 321bg overlap the support member 21ba in the Z direction parallel to the A8 axis and the A9 axis when the first lever member 321bb is closed with respect to the support member 21ba. Therefore, when the support member 21ba is rotated 180 degrees about the A7 axis with the first lever member 321bb closed with respect to the support member 21ba, the first switch 321bf is located at the position of the second switch 321bg, and the second switch 321bg is located at the position of the first switch 321bf. Thus, even when the support member 21ba is rotated 180 degrees about the A7 axis, the first switch 321bf and the second switch 321bg can be operated in the same manner.

In the second embodiment, as shown in FIGS. 16 to 19, the first lever member 321bb includes a plate-shaped attachment member 416 attached to an opposing surface 415 of the first lever member 321bb facing the support member 21ba to support the first switch 321bf and the second switch 321bg on support surfaces perpendicular to the opposing surface 415. The attachment member 416 moves integrally with the first lever member 321bb according to rotation of the first lever member 321bb. The attachment member 416 includes a first flat plate 416a parallel to the opposing surface 415, and two second flat plates 416b connected to the first flat plate 416a so as to be bent perpendicularly to the first flat plate 416a. The two second flat plates 416b are connected to a first side and a second side of the first flat plate 416a, respectively, in the Z direction parallel to the A8 axis and the A9 axis. Furthermore, the first switch 321bf and the second switch 321bg are attached to one and the other of the two second flat plates 416b, respectively, so as to be oriented in opposite directions. The two second flat plates 416b support the first switch 321bf and the second switch 321bg on the support surfaces perpendicular to the opposing surface 415. The second flat plates 416b extend from the first flat plate 416a toward the side adjacent to the support member 21ba. The attachment member 416 is not provided on the second lever member 321bc.

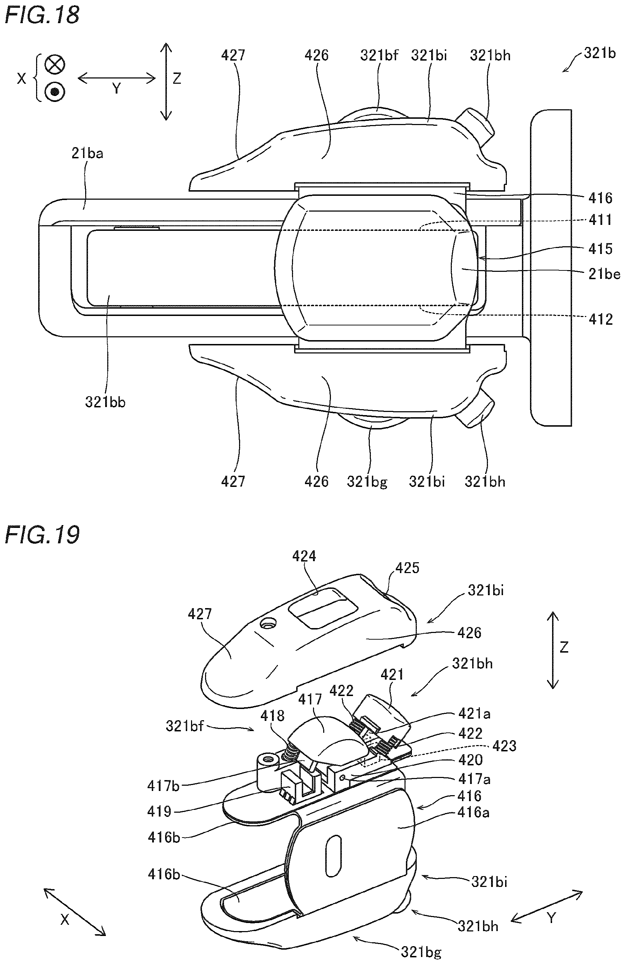

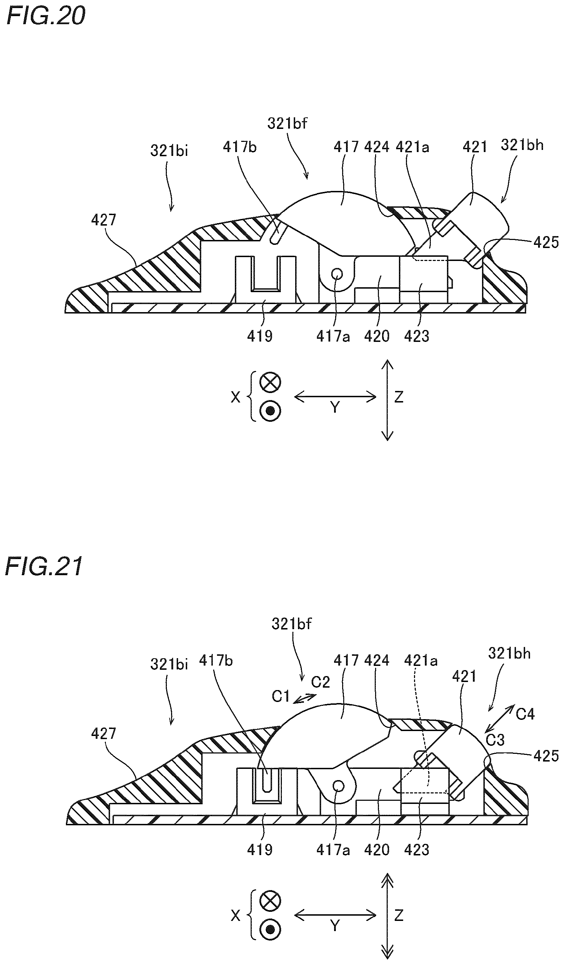

In the second embodiment, as shown in FIGS. 19 to 21, the first switch 321bf and the second switch 321bg are roller switches that are rotationally operated by the fingers of the operator. The first switch 321bf and the second switch 321bg as roller switches each include a rotary body 417 that is rotationally operated by the finger of the operator, an urging portion 418 that urges the rotary body 417 to an initial position (a position shown in FIG. 20), and a sensor 419 that detects movement of the rotary body 417. In FIGS. 20 and 21, only the first switch 321bf is shown for convenience.

The rotary body 417 has a fan shape and an operation surface curved in an arc. The operation surface of the rotary body 417 slightly protrudes from an opening 424 of a switch housing 321bi described below. Furthermore, the rotary body 417 is supported by a support 420 to which a rotation shaft 417a is attached via the rotation shaft 417a so as to rotate about the rotation shaft 417a. The rotary body 417 rotates about the rotation shaft 417a between the initial position (the position shown in FIG. 20) not detected by the sensor 419 and a detection position (a position shown in FIG. 21) detected by the sensor 419. The rotary body 417 includes a detected portion 417b that is detected by the sensor 419. The detected portion 417b has a convex shape protruding toward the sensor 419. The urging portion 418 is an elastic member that urges the rotary body 417 to the initial position. Specifically, the urging portion 418 is a compression coil spring. The sensor 419 is a transmissive photosensor including a light emitter and a light receiver.

As shown in FIGS. 20 and 21, when the operator operates the operation surface of the rotary body 417 by pulling it with his or her finger, the rotary body 417 is rotated to the detection position along a rotation direction C1 about the rotation shaft 417a while resisting the urging force of the urging portion 418. At this time, the detected portion 417b of the rotary body 417 is inserted between the light emitter and the light receiver of the sensor 419, and light from the light emitter to the light receiver is blocked. Then, the rotary body 417 is detected by the sensor 419. This state is an on-state. In the on-state, the clutch function is performed.

When the operation on the operation surface of the rotary body 417 by the operator is released, the rotary body 417 is rotated to the initial position along a rotation direction C2 opposite to the rotation direction C1 about the rotation shaft 417a due to the urging force of the urging portion 418. At this time, the detected portion 417b of the rotary body 417 is pulled out from between the light emitter and the light receiver of the sensor 419, and the blocking of the light from the light emitter to the light receiver is released such that passage of the light is allowed. Then, the rotary body 417 is no longer detected by the sensor 419. This state is an off-state. In the off-state, the clutch function is not performed.

In the second embodiment, as shown in FIGS. 16 to 18, the first switch 321bf and a third switch 321bh are arranged on the first lever member 321bb. The third switch 321bh is a customized switch in which a function to be performed by the customized switch is customized by function assignment to the customized switch. Functions related to the surgery of a patient P, such as clutch, incision, suction, drainage, image switching, and camera zooming, are assigned to the third switch 321bh. Function assignment to the third switch 321bh is described in detail below.

In the second embodiment, the third switch 321bh is inclined with respect to the first switch 321bf. Specifically, the third switch 321bh is inclined with respect to a Y direction that is the longitudinal direction of the support member 21ba. The third switch 321bh is positioned more distal than the first switch 321bf.

As shown in FIGS. 19 to 21, the third switch 321bh is a push-button switch that the operator operates by pressing it with his or her finger. The push-button third switch 321bh includes an operation body 421 that the operator operates by pressing it with his or her finger, urging portions 422 that urge the operation body 421 to the initial position (the position shown in FIG. 20), and a sensor 423 that detects movement of the operation body 421. Although FIGS. 20 and 21 show a state in which both the first switch 321bf and the third switch 321bh are operated for convenience, it is not necessary to operate both the first switch 321bf and the third switch 321bh at the same time. The first switch 321bf and the third switch 321bh can be operated independently of each other.

The operation body 421 has an operation surface inclined with respect to the first switch 321bf. The operation surface of the operation body 421 protrudes in an inclination direction from an opening 425 of the switch housing 321bi described below. Furthermore, the operation body 421 is supported so as to move in the inclination direction. The operation body 421 moves in the inclination direction between the initial position (the position shown in FIG. 20) not detected by the sensor 423 and the detection position (the position shown in FIG. 21) detected by the sensor 423. The operation body 421 includes a detected portion 421a that is detected by the sensor 423. The detected portion 421a has a convex shape protruding toward the sensor 423. The urging portions 422 are elastic members that urge the operation body 421 to the initial position. Specifically, the urging portions 422 are compression coil springs. The sensor 423 is a transmissive photosensor including a light emitter and a light receiver.

As shown in FIGS. 20 and 21, when the operator operates the operation surface of the operation body 421 by pressing it with his or her finger, the operation body 421 is linearly moved to the detection position along an inclination direction C3 while resisting the urging force of the urging portion 422. At this time, the detected portion 421a of the operation body 421 is inserted between the light emitter and the light receiver of the sensor 423, and light from the light emitter to the light receiver is blocked. Then, the operation body 421 is detected by the sensor 423. This state is an on-state. Then, the function having been assigned to the third switch 321bh is performed.

When the operation on the operation surface of the operation body 421 by the operator is released, the operation body 421 is linearly moved to the initial position along an inclination direction C4 opposite to the inclination direction C3 due to the urging force of the urging portion 422. At this time, the detected portion 421a of the operation body 421 is pulled out from between the light emitter and the light receiver of the sensor 423, and the blocking of the light from the light emitter to the light receiver is released such that passage of the light is allowed. Then, the operation body 421 is no longer detected by the sensor 423. This state is an off-state. Then, the function having been assigned to the third switch 321bh is not performed. In the third switch 321bh, the on-state and the off-state may be reversed. That is, in the off-state, the operation body 421 may be detected by the sensor 423, and in the on-state, the operation body 421 may not be detected by the sensor 423. Alternatively, the third switch 321bh may be switched between the on-state and the off-state each time the operation body 421 is detected by the sensor 423.

In the second embodiment, as shown in FIGS. 19 to 21, the third switch 321bh and the first switch 321bf are arranged in one switch housing 321bi. The switch housing 321bi includes a finger rest on which the fingers of the operator are placed. The switch housing 321bi is arranged on the first lever member 321bb that is operated by the index finger or middle finger of the operator. That is, in the present embodiment, the remote control apparatus 302 includes the finger rest attached to the first lever member 321bb and on which the fingers of the operator are placed. The switch housing 321bi is an example of a housing.

The switch housing 321bi covers the first switch 321bf and the third switch 321bh from the first side in the Z direction parallel to the A8 axis and the A9 axis. The switch housing 321bi has a shape extending in the Y direction that is the longitudinal direction of the support member 21ba. The switch housing 321bi includes the opening 424 in which the first switch 321bf is arranged, and the opening 425 in which the third switch 321bh is arranged.

The switch housing 321bi has a side surface portion 426 and an inclined surface portion 427 as portions that function as finger rests. The side surface portion 426 is a side surface portion arranged on the first lever member 321bb side of the switch housing 321bi in an X direction, and extends in the Z direction parallel to the A8 axis and the A9 axis and in the Y direction that is the longitudinal direction of the support member 21ba. The operator can rest the index finger of the right hand that is not operating the first lever member 321bb by placing it on the side surface portion 426 from the side while placing the middle finger of the right hand on the first lever member 321bb. The inclined surface portion 427 is positioned more proximal than the openings 424 and 425 in the Y direction, and is inclined with respect to the Y direction. Specifically, the inclined surface portion 427 is inclined so as to be depressed from the first side to the second side in the Z direction. The operator can rest the index finger of the right hand that is not operating the first lever member 321bb by placing it on the inclined surface portion 427 from the first side in the Z direction while placing the middle finger of the right hand on the first lever member 321bb. Both the side surface portion 426 and the inclined surface portion 427 are portions of the switch housing 321bi in which the first switch 321bf and the third switch 321bh are not arranged, and even when the operator places and rests his or her fingers on the side surface portion 426 and the inclined surface portion 427, there is no risk of erroneously operating the first switch 321bf and the third switch 321bh.

A third switch 321bh is also arranged for the second switch 321bg. The configurations of the third switch 321bh and a switch housing 321bi regarding the second switch 321bg are the same as or similar to the configurations of the third switch 321bh and the switch housing 321bi regarding the first switch 321bf.

The operator can place and rest his or her fingers on the switch housing 321bi arranged for the second switch 321bg, as described below. That is, the operator can rest the ring finger of the right hand that is not operating the first lever member 321bb by placing it on a side surface portion 426 from the side while placing the middle finger of the right hand on the first lever member 321bb. Furthermore, the operator can rest the middle finger of the right hand that is not operating the first lever member 321bb by placing it on the side surface portion 426 from the side while placing the index finger of the right hand on the first lever member 321bb. Similarly, the operator can rest the ring finger of the right hand that is not operating the first lever member 321bb by placing it on an inclined surface portion 427 from the second side in the Z direction while placing the middle finger of the right hand on the first lever member 321bb. Furthermore, the operator can rest the middle finger of the right hand that is not operating the first lever member 321bb by placing it on the inclined surface portion 427 from the second side in the Z direction while placing the index finger of the right hand on the first lever member 321bb.

Although the first switch 321bf, the second switch 321bg, and the third switch 321bh provided on the operation handle 321b of the right-hand operation manipulator 21R have been described, the operation handle 321b of the left-hand operation manipulator 21L has the same or similar structure as that of the operation handle 321b of the right-hand operation manipulator 21R, except that the operation handle 321b of the left-hand operation manipulator 21L has a structure bilaterally symmetrical to that of the operation handle 321b of the right-hand operation manipulator 21R. A manipulator arm 60 operated by the right-hand operation manipulator 21R and a manipulator arm 60 operated by the left-hand operation manipulator 21L may be clutched independently of each other by the first switch 321bf of the right-hand operation manipulator 21R and the first switch 321bf of the left-hand operation manipulator 21L. That is, when the first switch 321bf of the right-hand operation manipulator 21R is operated, the manipulator arm 60 operated by the right-hand operation manipulator 21R may be clutched, and when the first switch 321bf of the left-hand operation manipulator 21L is operated, the manipulator arm 60 operated by the left-hand operation manipulator 21L may be clutched.

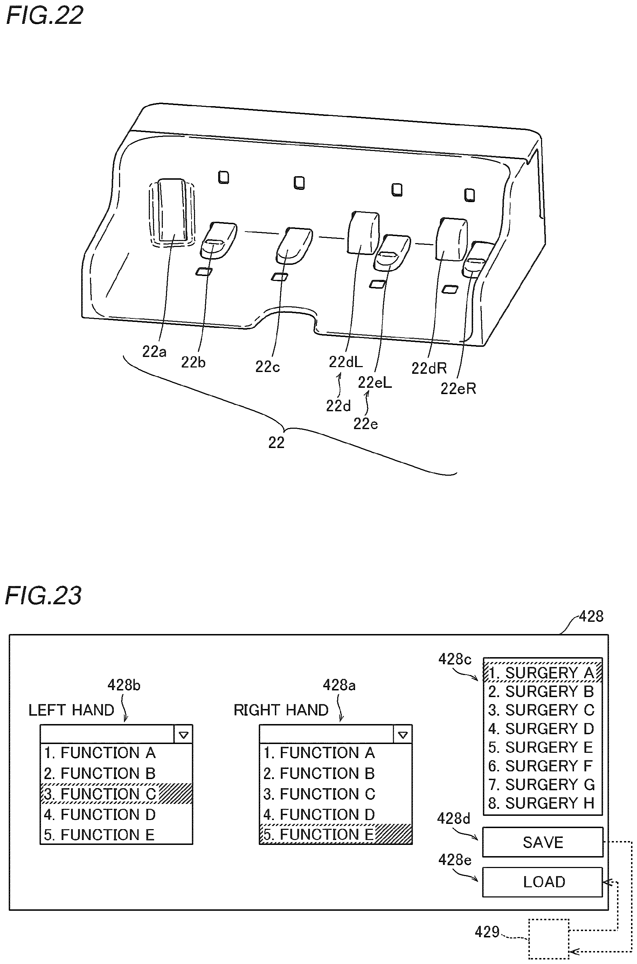

Operation pedals 22 operated by the foot of the operator are now described with reference to FIG. 22. A plurality of operation pedals 22 are provided to perform functions related to a surgical instrument 4. The operation pedals 22 include a switching pedal 22a, a clutch pedal 22b, a camera pedal 22c, an incision pedal 22d, and a coagulation pedal 22e. The switching pedal 22a, the clutch pedal 22b, the camera pedal 22c, the incision pedal 22d, and the coagulation pedal 22e are operated by the foot of the operator. The incision pedal 22d includes an incision pedal 22dR for a right manipulator arm 60, and an incision pedal 22dL for a left manipulator arm 60. The coagulation pedal 22e includes a coagulation pedal 22eR for the right manipulator arm 60 and a coagulation pedal 22eL for the left manipulator arm 60. The camera pedal 22c is an example of a foot pedal.

The switching pedal 22a switches the manipulator arms 60 to be operated by the operation handles 21b. The clutch pedal 22b performs a clutch function of preventing an operation of the operator from being transmitted to the medical manipulator 1. While the clutch pedal 22b is being pressed by the operator, operations by the operation handles 21b are not transmitted to the manipulator arms 60. While the camera pedal 22c is being pressed by the operator, the operation unit 21b can operate the manipulator arm 60 to which an endoscope 4b is attached. While the incision pedal 22d or the coagulation pedal 22e is being pressed by the operator, an electrosurgical device is activated.

In the second embodiment, the first switch 321bf performs a function different from the clutch function that is a predetermined function, by being operated simultaneously with the camera pedal 22c. Specifically, the first switch 321bf performs a function related to an image captured by the endoscope 4b by being operated simultaneously with the camera pedal 22c. When the first switch 321bf is operated simultaneously with the camera pedal 22c, the first switch 321bf performs different functions depending on how the first switch 321bf is operated. More specifically, when the first switch 321bf is operated simultaneously with the camera pedal 22c, the first switch 321bf performs different functions depending on at least one of the operation time of the first switch 321bf or the number of operations of the first switch 321bf.

For example, when the first switch 321bf is operated simultaneously with the camera pedal 22c, the first switch 321bf performs a function of switching the image captured by the endoscope 4b when a long press operation is performed in which the operation time of the first switch 321bf is equal to or longer than a predetermined time. For example, in a surgery performed by administering indocyanine green (ICG), which is a fluorescent substance, to the patient P, an ICG fluorescence image and a normal image that is not an ICG fluorescence image are switched.

For example, when the first switch 321bf is operated simultaneously with the camera pedal 22c, the first switch 321bf performs a function of zooming in on the image captured by the endoscope 4b when a short press operation is performed in which the operation time of the first switch 321bf is less than the predetermined time. Furthermore, for example, when the first switch 321bf is operated simultaneously with the camera pedal 22c, the first switch 321bf performs a function of zooming out of the image captured by the endoscope 4b when a short press operation is performed twice in which the operation time of the first switch 321bf is less than the predetermined time.

The right-hand first switch 321bf and the left-hand first switch 321bf may perform different functions when the camera pedal 22c is operated simultaneously. For example, when the camera pedal 22c and the right-hand first switch 321bf are operated simultaneously, the right-hand first switch 321bf may perform the function of zooming in on the image captured by the endoscope 4b, and when the camera pedal 22c and the left-hand first switch 321bf are operated simultaneously, the left-hand first switch 321bf may perform the function of zooming out of the image captured by the endoscope 4b.

Function assignment to the third switch 321bh is now described with reference to FIG. 23. As shown in FIG. 23, a setting screen 428 is displayed on a touch panel 23 or a monitor 24, for example, when a function is assigned to the third switch 321bh. The setting screen 428 includes function selection fields 428a and 428b, a surgery name selection field 428c, a save button 428d, and a load button 428e.

The function selection field 428a is a field for selecting a function to be assigned to the right-hand third switch 321bh. In the function selection field 428a, a plurality of functions to be assigned to the right-hand third switch 321bh are selectably displayed in a pull-down format. An arbitrary function selected by the operator from among the plurality of functions displayed in a pull-down format in the function selection field 428a is assigned to the right-hand third switch 321bh.

The function selection field 428b is a field for selecting a function to be assigned to the left-hand third switch 321bh. In the function selection field 428b, a plurality of functions to be assigned to the left-hand third switch 321bh are selectably displayed in a pull-down format. An arbitrary function selected by the operator from among the plurality of functions displayed in a pull-down format in the function selection field 428b is assigned to the left-hand third switch 321bh.

The surgery name selection field 428c is a field for selecting a surgery name. In the surgery name selection field 428c, a plurality of surgery names are selectably displayed. The function of the right-hand third switch 321bh selected in the function selection field 428a and the function of the left-hand third switch 321bh selected in the function selection field 428b are stored in a storage 429 of the remote control apparatus 302 in association with an arbitrary surgery name selected by the operator from among the plurality of surgery names displayed in the surgery name selection field 428c. Thus, the functions of the right-hand third switch 321bh and the left-hand third switch 321bh can be managed for each surgery name displayed in the surgery name selection field 428c. The storage 429 is a nonvolatile storage device such as a flash memory.

The save button 428d is a button on the display for the operator to save settings. When the save button 428d is operated, the function of the right-hand third switch 321bh selected in the function selection field 428a and the function of the left-hand third switch 321bh selected in the function selection field 428b are stored as data to be saved in the storage 429 in association with the surgery name selected in the surgery name selection field 428c. Furthermore, the remote control apparatus 302 reads last used saved data from the storage 429 when the power is turned on. Thus, when the last used saved data is used, it is not necessary to reset the function of the third switch 321bh.

The load button 428e is a button on the display for the operator to read the settings. When the load button 428e is operated with a surgery name selected in the surgery name selection field 428c, the saved data corresponding to the surgery name selected in the surgery name selection field 428c is read from the storage 429. Then, the function of the right-hand third switch 321bh and the function of the left-hand third switch 321bh, which are associated with the surgery name selected in the surgery name selection column 428c, are set as the functions of the switches.

The remaining configurations of the second embodiment are similar to those of the first embodiment.

### Advantages of Second Embodiment

According to the second embodiment, the following advantages are achieved.

According to the second embodiment, as described above, the remote control apparatus 302 includes the first switch 321bf attached to the first lever member 321bb to perform the predetermined function. Accordingly, similarly to the first embodiment, when the operator of the remote control apparatus 302 operates the medical manipulator 1 including the manipulator arm 60 having a tip end to which the surgical instrument 4 is attached, the operability of the first switch 21bf operated by the hand of the operator can be improved.

According to the second embodiment, as described above, the first switch 321bf is a roller switch. Accordingly, the operator can rotationally operate the first switch 321bf as a roller switch, and thus the operator can easily operate the first switch 321bf as a roller switch.

According to the second embodiment, as described above, the roller switch includes the rotary body 417 rotationally operated by the operator, the urging portion 418 to urge the rotary body 417 to the initial position, and the sensor 419 to detect movement of the rotary body 417. Accordingly, when the operator operates the rotary body 417, movement of the rotary body 417 can be detected by the sensor 419. When the operator does not operate the rotary body 417 or when the operator releases his or her finger after the operation, the urging portion 418 urges the rotary body 417 to the initial position such that the sensor 419 cannot detect movement of the rotary body 417.

According to the second embodiment, as described above, the first switch 321bf and the third switch 321bh are arranged on the first lever member 321bb. Accordingly, the first switch 321bf and the third switch 321bh are arranged on the first lever member 321bb, and thus the operator can selectively use the first switch 321bf and the third switch 321bh.

According to the second embodiment, as described above, the third switch 321bh is inclined with respect to the first switch 321bf. Accordingly, the third switch 321bh can be operated from a direction inclined with respect to the first switch 321bf, and thus erroneous pressing between the third switch 321bh and the first switch 321bf can be effectively reduced or prevented.

According to the second embodiment, as described above, the remote control apparatus 302 includes the camera pedal 22c operated by the foot of the operator. Furthermore, the first switch 321bf is operable to perform the function different from the predetermined function by being operated simultaneously with the camera pedal 22c. Accordingly, the predetermined function can be performed by the first switch 321bf, and the function different from the predetermined function can also be performed by simultaneously operating the first switch 321bf and the camera pedal 22c. Consequently, the first switch 321bf can be effectively used to perform various functions.

According to the second embodiment, as described above, the first switch 321bf is operable to perform different functions depending on how the first switch 321bf is operated when the first switch 321bf is operated simultaneously with the camera pedal 22c. Accordingly, different functions can be performed depending on how the first switch 321bf is operated when the first switch 321bf and the camera pedal 22c are operated simultaneously, and thus the first switch 321bf can be more effectively used to perform more various functions.

According to the second embodiment, as described above, the first switch 321bf is operable to perform different functions depending on at least one of the operation time of the first switch 321bf or the number of operations of the first switch 321bf when the first switch 321bf is operated simultaneously with the camera pedal 22c. Accordingly, different functions can be easily performed depending on at least one of the operation time of the first switch 321bf or the number of operations of the first switch 321bf when the first switch 321bf and the camera pedal 22c are operated simultaneously.

According to the second embodiment, as described above, the first lever member 321bb further includes the plate-shaped attachment member 416 attached to the opposing surface 415 of the first lever member 321bb facing the support member 21ba to support the first switch 321bf perpendicularly to the opposing surface 415. Accordingly, as compared with a case in which the first switch 321bf is arranged parallel to the opposing surface 415 of the first lever member 321bb facing the support member 21ba, the first switch 321bf can be easily operated, and thus the operability of the switch 321bf can be improved.

According to the second embodiment, as described above, the remote control apparatus 302 includes the switch housing 321bi in which the first switch 321bf is arranged. Furthermore, the switch housing 321bi includes a finger rest on which the fingers of the operator are placed. Accordingly, the operator can rest his or her fingers by placing them on the switch housing 321bi as a finger rest, and thus fatigue on the fingers of the operator can be mitigated. Furthermore, the switch housing 321bi in which the first switch 321bf is arranged is used as a finger rest, and thus an increase in the number of components can be reduced or prevented as compared with a case in which a finger rest is provided separately from the switch housing 321bi.

According to the second embodiment, as described above, the switch housing 321bi is arranged on the first lever member 321bb operated by the index finger or middle finger of the operator. When the operator operates the first lever member 321bb with his or her index finger, the middle finger and the ring finger, for example, other than the index finger become the fingers of the operator that can be rested on the finger rest. When the operator operates the first lever member 321bb with his or her middle finger, the index finger and the ring finger, for example, other than the middle finger become the fingers of the operator that can be rested on the finger rest. Therefore, with the configuration described above, the switch housing 321bi is arranged on the first lever member 321bb on the side on which the fingers of the operator that can be rested, other than the finger (index finger or middle finger) of the operator operating the first lever member 321bb, are present, and thus the switch housing 321bi can be effectively used as a finger rest.

According to the second embodiment, as described above, the first switch 321bf and the second switch 321bg operable to perform the predetermined function are arranged on the first lever member 321bb. Accordingly, the first switch 321bf and the second switch 321bg are arranged on the first lever member 321bb, and thus as compared with a case in which the second switch 321bg is arranged on a member other than the first lever member 321bb, the complexity of the structure can be reduced or prevented, and the first switch 321bf and the second switch 321bg can be easily arranged.

The remaining advantages of the second embodiment are similar to those of the first embodiment.

### Third Embodiment

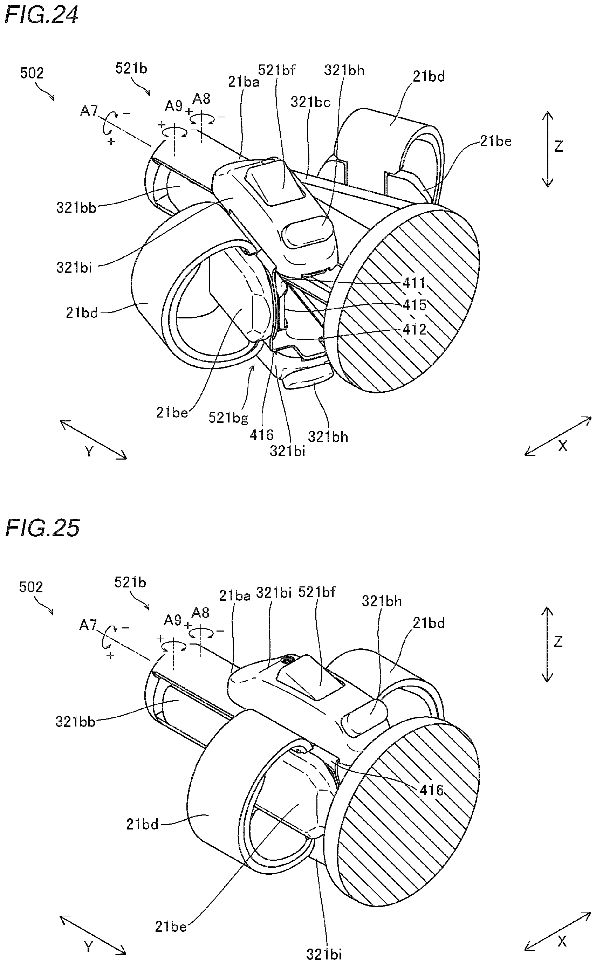

A remote control apparatus 502 according to a third embodiment is now described with reference to FIGS. 24 to 29. The same or similar configurations as those of the first embodiment are denoted by the same reference numerals, and detailed description thereof is omitted. The remote control apparatus 502 is an example of an operator-side apparatus.

As shown in FIGS. 24 and 25, the remote control apparatus 502 according to the third embodiment includes operation handles 521b in place of the operation handles 321b according to the second embodiment. The operation handle 521b of a right-hand operation manipulator 21R is described below. The configurations of a left-hand operation manipulator 21L and the right-hand operation manipulator 21R are the same as or similar to each other except that the operation handles 521b thereof have bilaterally symmetrical structures.

In the third embodiment, as shown in FIGS. 24 to 26, the operation handle 521b includes a first switch 521bf and a second switch 521bg in place of the first switch 321bf and the second switch 321bg according to the second embodiment. The first switch 521bf and the second switch 521bg are attached to a first lever member 321bb to perform predetermined functions. Specifically, the first switch 521bf is a clutch switch that performs a clutch function of preventing an operation of an operator from being transmitted to a medical manipulator 1. The first switch 521bf and the second switch 521bg have the same or similar configurations as those of the first switch 321bf and the second switch 321bg according to the second embodiment, except for switch structures. Therefore, the switch structures of the first switch 521bf and the second switch 521bg are described in detail.

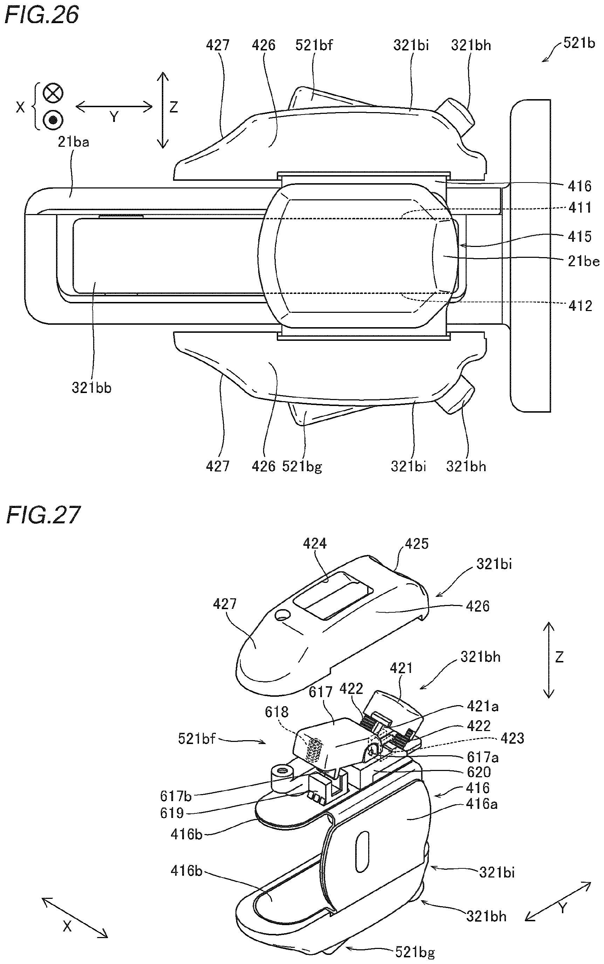

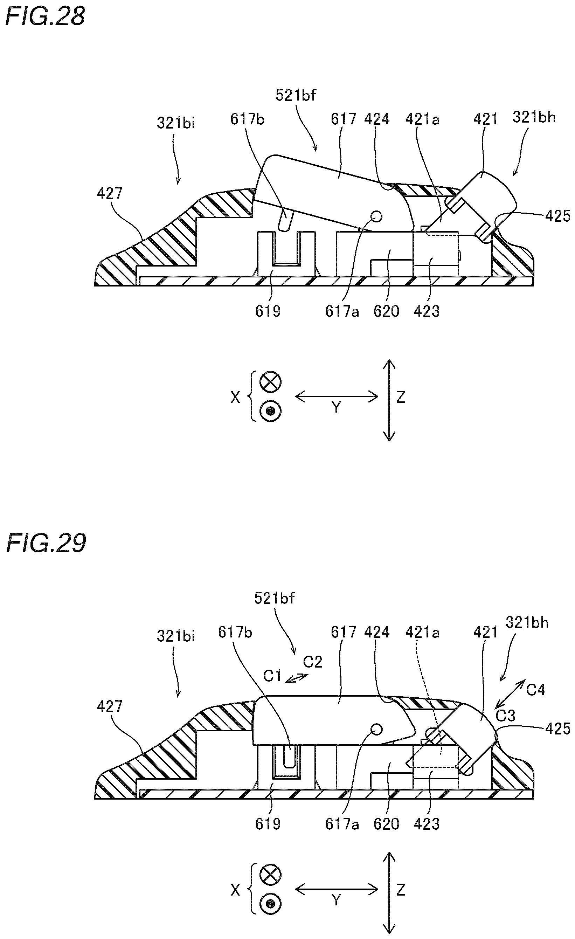

In the third embodiment, as shown in FIGS. 27 to 29, the first switch 521bf and the second switch 521bg are seesaw switches that the operator operates by pressing them with his or her fingers. The first switch 521bf and the second switch 521bg as seesaw switches each include a seesaw body 617 that the operator operates by pressing it with his or her finger, an urging portion 618 that urges the seesaw body 617 to an initial position (a position shown in FIG. 28), and a sensor 619 that detects movement of the seesaw body 617. In FIGS. 28 and 29, only the first switch 521bf is shown for convenience.

The seesaw body 617 has a rectangular parallelepiped shape and a flat operation surface. The operation surface of the seesaw body 617 protrudes from an opening 424 of a switch housing 321bi such that the proximal end thereof is higher than the distal end thereof. The seesaw body 617 is supported by a support 620 to which a rotation shaft 617a is attached via the rotation shaft 617a so as to rotate about the rotation shaft 617a. The rotation shaft 617a is arranged at the distal end of the seesaw body 617. The seesaw body 617 rotates about the rotation axis 617a between the initial position (the position shown in FIG. 28) not detected by the sensor 619 and a detection position (a position shown in FIG. 29) detected by the sensor 619. The seesaw body 617 includes a detected portion 617b that is detected by the sensor 619. The detected portion 617b has a convex shape protruding toward the sensor 619. The urging portion 618 is an elastic member that urges the seesaw body 617 to the initial position. Specifically, the urging portion 618 is a compression coil spring. The sensor 619 is a transmissive photosensor including a light emitter and a light receiver.

As shown in FIGS. 28 and 29, when the operator operates the operation surface of the seesaw body 617 by pressing it with his or her finger, the seesaw body 617 is rotated to the detection position along a rotation direction C1 about the rotation shaft 617a while resisting the urging force of the urging portion 618. At this time, the detected portion 617b of the seesaw body 617 is inserted between the light emitter and the light receiver of the sensor 619, and light from the light emitter to the light receiver is blocked. Then, the seesaw body 617 is detected by the sensor 619. This state is an on-state. In the on-state, the clutch function is performed.

When the operation on the operation surface of the seesaw body 617 by the operator is released, the seesaw body 617 is rotated to the initial position along a rotation direction C2 opposite to the rotation direction C1 about the rotation shaft 617a due to the urging force of the urging portion 618. At this time, the detected portion 617b of the seesaw body 617 is pulled out from between the light emitter and the light receiver of the sensor 619, and the blocking of the light from the light emitter to the light receiver is released such that passage of the light is allowed. Then, the seesaw body 617 is no longer detected by the sensor 619. This state is an off-state. In the off-state, the clutch function is not performed.

Although the first switch 521bf and the second switch 521bg provided on the operation handle 521b of the right-hand operation manipulator 21R have been described above, the operation handle 521b of the left-hand operation manipulator 21L has the same or similar structure as that of the operation handle 521b of the right-hand operation manipulator 21R, except that the operation handle 521b of the left-hand operation manipulator 21L has a structure bilaterally symmetrical to that of the operation handle 521b of the right-hand operation manipulator 21R.

The remaining configurations of the third embodiment are similar to those of the second embodiment.

### Advantages of Third Embodiment

According to the third embodiment, the following advantages are achieved.

According to the third embodiment, as described above, the remote control apparatus 502 includes the first switch 521bf attached to the first lever member 321bb to perform the predetermined function. Accordingly, similarly to the first embodiment, when the operator of the remote control apparatus 302 operates the medical manipulator 1 including a manipulator arm 60 having a tip end to which a surgical instrument 4 is attached, the operability of the first switch 521bf operated by the hand of the operator can be improved.

According to the third embodiment, as described above, the first switch 521bf is a seesaw switch. Accordingly, the operator can operate the first switch 521bf as a seesaw switch by pressing it, and thus the operator can easily operate the first switch 521bf as a seesaw switch.

According to the third embodiment, as described above, the seesaw switch includes the seesaw body 617 operated by being pressed by the finger of the operator, the urging portion 618 to urge the seesaw body 617 to the initial position, and the sensor 619 to detect movement of the seesaw body 617. Accordingly, when the operator operates the seesaw body 617, movement of the seesaw body 617 can be detected by the sensor 619. When the operator does not operate the seesaw body 617 or when the operator releases his or her finger after the operation, the urging portion 618 urges the seesaw body 617 to the initial position such that the sensor 619 cannot detect movement of the seesaw body 617.

The remaining advantages of the third embodiment are similar to those of the second embodiment.

### Modified Examples

The embodiments disclosed this time must be considered as illustrative in all points and not restrictive. The scope of the present disclosure is not shown by the above description of the embodiments but by the scope of claims for patent, and all modifications (modified examples) within the meaning and scope equivalent to the scope of claims for patent are further included.

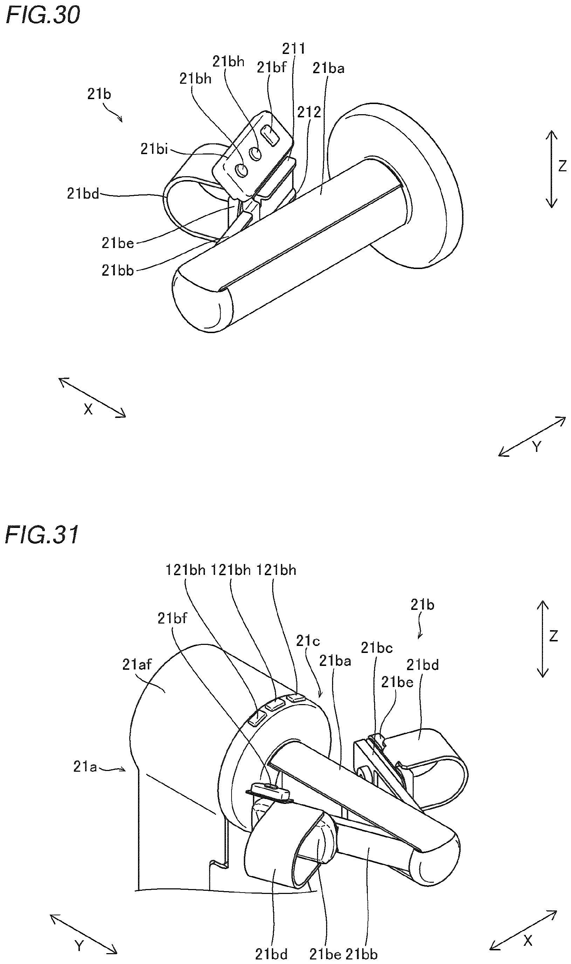

For example, while the example in which the operation handle includes the first lever member and the second lever member has been shown in each of the aforementioned first to third embodiments, the present disclosure is not limited to this. For example, as in a first modified example shown in FIG. 30, an operation handle 21b may include only a first lever member 21bb.

While the example in which the first switch is attached to the first lever member, and the second switch is attached to the second lever member has been shown in the aforementioned first embodiment, and the example in which the first switch and the second switch are attached to the first lever member has been shown in each of the aforementioned second and third embodiments, the present disclosure is not limited to this. For example, the first switch may be attached to the first lever member, and the second switch may not be attached to the first lever member or the second lever member.

While the example in which the first switch is arranged at the first end of the first lever member, and the second switch is arranged at the second end of the second lever member has been shown in the aforementioned first embodiment, and the example in which the first switch is arranged at the first end of the first lever member, and the second switch is arranged at the second end of the first lever member has been shown in each of the aforementioned second and third embodiments, the present disclosure is not limited to this. For example, the first switch may be arranged at the second end of the first lever member or may be arranged at the first and second ends of the first lever member. Furthermore, the second switch may be arranged at the first end of the second lever member or may be arranged at the first and second ends of the second lever member.

While the example in which the first switch and the second switch are clutch switches has been shown in each of the aforementioned first to third embodiments, the present disclosure is not limited to this. For example, the first switch and the second switch may be switches that perform functions other than the clutch function.

While the example in which the first switch and the second switch are supported so as to be inclined with respect to the opposing surface has been shown in the aforementioned first embodiment, and the example in which the first switch and the second switch are supported by the support surfaces perpendicular to the opposing surface has been shown in each of the aforementioned second and third embodiments, the present disclosure is not limited to this. For example, the first switch and the second switch may be supported by support surfaces parallel to the opposing surface.

While the example in which the first switch and the second switch are push-button has been shown in the aforementioned first embodiment, the example in which the first switch and the second switch are roller switches has been shown in the aforementioned second embodiment, and the example in which the first switch and the second switch are seesaw switches has been shown in the aforementioned third embodiment, the present disclosure is not limited to this. For example, the first switch and the second switch may be slide switches other than push-button switches, roller switches, and seesaw switches.

While the example in which the first switch and the second switch are redundant switches has been shown in the aforementioned first embodiment, the present disclosure is not limited to this. For example, the first switch and the second switch may not be redundant switches.

While the example in which the remote control apparatus includes the customized switches has been shown in each of the aforementioned first to third embodiments, the present disclosure is not limited to this. For example, the remote control apparatus may not include the customized switches.

While the example in which the customized switches are arranged on the first lever member and the second lever member has been shown in the aforementioned first embodiment, and the example in which the customized switches are arranged on the first lever member has been shown in each of the aforementioned second and third embodiments, the present disclosure is not limited to this.

For example, as in a second modified example shown in FIG. 31, a plurality of (three) customized switches 121bh may be arranged on a flange 21c connecting the proximal end of an operation arm 21a to a support member 21ba. Accordingly, the plurality of customized switches 121bh can be arranged on the flange 21c on which it is relatively easy to ensure a space for arranging the plurality of customized switches 121bh, and thus the plurality of customized switches 121bh can be easily arranged. In the second modified example shown in FIG. 31, the plurality of customized switches 121bh are arranged at an upper position on the outer peripheral surface of the flange 21c, but the arrangement positions of the plurality of customized switches 121bh are not particularly limited. The plurality of customized switches 121bh may be arranged at a lower position on the outer peripheral surface of the flange 21c or may be arranged at a side position on the outer peripheral surface of the flange 21c.

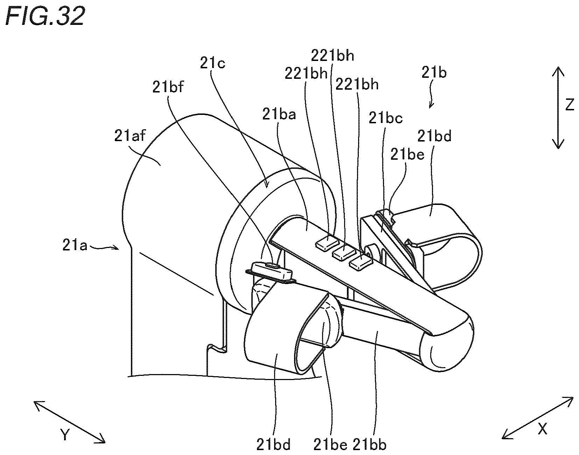

Alternatively, for example, as in a third modified example shown in FIG. 32, a plurality of (three) customized switches 221bh may be arranged on a support member 21ba. Accordingly, functions having been assigned to the plurality of customized switches 221bh can be performed by operations by the hand of an operator.

While the example in which the first switch arranged on the first lever member performs the function different from the predetermined function by being operated simultaneously with the foot pedal has been shown in each of the aforementioned second and third embodiments, the present disclosure is not limited to this. For example, a switch arranged on other than the first lever member to perform the predetermined function may perform the function different from the predetermined function by being operated simultaneously with the foot pedal.

While the example in which the first switch arranged on the first lever member is a roller switch has been shown in the aforementioned second embodiment, and the example in which the first switch arranged on the first lever member is a seesaw switch has been shown in the aforementioned third embodiment, the present disclosure is not limited to this. For example, a switch arranged on other than the first lever member to perform the predetermined function may be a roller switch or a seesaw switch.

While the example in which the housing in which the first switch is arranged includes a finger rest has been shown in each of the aforementioned second and third embodiments, the present disclosure is not limited to this. For example, the switch may not be arranged on the finger rest.

While the example in which the first switch and the third switch as a customized switch are arranged on the first lever member has been shown in each of the aforementioned second and third embodiments, the present disclosure is not limited to this. For example, the first switch and the third switch as a switch other than a customized switch may be arranged on the first lever member.

While the example in which the foot pedal operated simultaneously with the first switch is the camera pedal has been shown in each of the aforementioned second and third embodiments, the present disclosure is not limited to this. For example, the foot pedal operated simultaneously with the first switch may be other than the camera pedal.

### Aspects

It will be appreciated by those skilled in the art that the exemplary embodiments described above are specific examples of the following aspects.

### (Item 1)

An operator-side apparatus operable to operate a patient-side apparatus including a patient-side arm having a tip end to which a surgical instrument is attached, the operator-side apparatus comprising:
an operation arm;
a support member connected to a proximal end of the operation arm;
a first lever member attached to the support member so as to rotate with respect to the support member; and
a first switch attached to the first lever member to perform a predetermined function.

### (Item 2)

The operator-side apparatus according to item 1, wherein
a distal end of the support member is connected to the proximal end of the operation arm so as to rotate about a first axis parallel to a longitudinal direction of the support member; and
a proximal end of the first lever member is attached to the support member so as to rotate about a second axis perpendicular to the first axis with respect to the support member.

### (Item 3)

The operator-side apparatus according to item 2, wherein the first switch is arranged closer to a distal end than the proximal end of the first lever member.

### (Item 4)

The operator-side apparatus according to item 3, wherein the first switch is arranged at at least one of a first end or a second end of the first lever member in a direction parallel to the second axis.

### (Item 5)

The operator-side apparatus according to any one of items 1 to 4, comprising:
a second lever member facing the first lever member via the support member; and
a second switch attached to the second lever member to perform the predetermined function; wherein
a distal end of the support member is connected to the proximal end of the operation arm so as to rotate about a first axis parallel to a longitudinal direction of the support member; and
a proximal end of the second lever member is attached to the support member so as to rotate about a third axis perpendicular to the first axis with respect to the support member.

### (Item 6)

The operator-side apparatus according to item 5, wherein the second switch is arranged closer to a distal end than the proximal end of the second lever member.

### (Item 7)

The operator-side apparatus according to item 5 or 6, wherein the second switch is arranged at at least one of a first end or a second end of the second lever member in a direction parallel to the third axis.

### (Item 8)

The operator-side apparatus according to any one of items 1 to 7, wherein the first switch includes a clutch switch to perform a clutch function of preventing an operation of an operator from being transmitted to the patient-side apparatus.

### (Item 9)

The operator-side apparatus according to any one of items 5 to 7, wherein the first switch and the second switch are arranged symmetrically about the first axis of the support member.

### (Item 10)

The operator-side apparatus according to any one of items 1 to 9, wherein the first switch is inclined with respect to an opposing surface of the first lever member facing the support member.

### (Item 11)

The operator-side apparatus according to item 10, wherein
the first lever member further includes a plate-shaped attachment member attached to the opposing surface to support the first switch such that the first switch is inclined with respect to the opposing surface;
the attachment member includes a first flat plate parallel to the opposing surface, and a second flat plate connected to the first flat plate and inclined so as to be bent with respect to the first flat plate; and
the first switch is attached to the second flat plate.

### (Item 12)

The operator-side apparatus according to any one of items 1 to 11, wherein the first switch is push-button.

### (Item 13)

The operator-side apparatus according to item 12, wherein the push-button first switch is a clutch switch operable to perform a clutch function of preventing an operation of an operator from being transmitted to the patient-side apparatus, and is a redundant switch.

### (Item 14)

The operator-side apparatus according to any one of items 1 to 11, wherein the first switch is a roller switch.

### (Item 15)

The operator-side apparatus according to item 14, wherein the roller switch includes a rotary body rotationally operated by an operator, an urging portion to urge the rotary body to an initial position, and a sensor to detect movement of the rotary body.

### (Item 16)

The operator-side apparatus according to any one of items 1 to 11, wherein the first switch is a seesaw switch.

### (Item 17)

The operator-side apparatus according to item 16, wherein the seesaw switch includes a seesaw body operated by being pressed by a finger of an operator, an urging portion to urge the seesaw body to an initial position, and a sensor to detect movement of the seesaw body.

### (Item 18)

The operator-side apparatus according to any one of items 1 to 17, further comprising:
a customized switch to perform a function other than a clutch function of preventing an operation of an operator from being transmitted to the patient-side apparatus; wherein
a function to be performed by the customized switch is customized by function assignment to the customized switch.

### (Item 19)

The operator-side apparatus according to item 18, wherein the customized switch is arranged on the first lever member.

### (Item 20)

The operator-side apparatus according to item 18, wherein the customized switch is arranged on the support member or a flange connecting the proximal end of the operation arm to the support member.

### (Item 21)

The operator-side apparatus according to any one of items 1 to 17, wherein the first switch and a third switch are arranged on the first lever member.

### (Item 22)

The operator-side apparatus according to item 21, wherein the third switch is inclined with respect to the first switch.

### (Item 23)

The operator-side apparatus according to any one of items 1 to 22, further comprising:
a foot pedal operated by a foot of an operator; wherein
the first switch is operable to perform a function different from the predetermined function by being operated simultaneously with the foot pedal.

### (Item 24)

The operator-side apparatus according to item 23, wherein the first switch is operable to perform different functions depending on how the first switch is operated when the first switch is operated simultaneously with the foot pedal.

### (Item 25)

The operator-side apparatus according to item 24, wherein the first switch is operable to perform different functions depending on at least one of an operation time of the first switch or a number of operations of the first switch when the first switch is operated simultaneously with the foot pedal.

### (Item 26)

The operator-side apparatus according to any one of items 1 to 9 and 12 to 25, wherein the first lever member further includes a plate-shaped attachment member attached to an opposing surface of the first lever member facing the support member to support the first switch on a support surface perpendicular to the opposing surface.

### (Item 27)

The operator-side apparatus according to any one of items 1 to 26, further comprising:
an angle sensor to detect an angle of the first lever member with respect to the support member;
an FPC in which the angle sensor and the first switch are arranged; and
a reinforcing plate arranged at a position of the angle sensor of the FPC.

### (Item 28)

The operator-side apparatus according to any one of items 1 to 27, further comprising:
a housing in which the first switch is arranged; wherein
the housing includes a finger rest on which a finger of an operator is placed.

### (Item 29)

The operator-side apparatus according to item 28, wherein the housing is arranged on the first lever member operated by an index finger or middle finger of the operator.

### (Item 30)

The operator-side apparatus according to any one of items 1 to 4, 8, and 10 to 29, wherein the first switch and a second switch operable to perform the predetermined function are arranged on the first lever member.

### (Item 31)

An operator-side apparatus operable to operate a patient-side apparatus including a patient-side arm having a tip end to which a surgical instrument is attached, the operator-side apparatus comprising:
an operation arm;
a support member connected to a proximal end of the operation arm;
a lever member attached to the support member so as to rotate with respect to the support member; and
a finger rest attached to the lever member and on which a finger of an operator is placed.

### (Item 32)

A robotic surgical system comprising:
a patient-side apparatus including a patient-side arm having a tip end to which a surgical instrument is attached; and
an operator-side apparatus operable to operate the patient-side apparatus; wherein
the operator-side apparatus includes:
   an operation arm;
   a support member connected to a proximal end of the operation arm;
   a lever member attached to the support member so as to rotate with respect to the support member; and
   a switch attached to the lever member to perform a predetermined function.

### (Item 33)

A robotic surgical system comprising:
a patient-side apparatus including a patient-side arm having a tip end to which a surgical instrument is attached; and
an operator-side apparatus operable to operate the patient-side apparatus; wherein
the operator-side apparatus includes:
   an operation arm;
   a support member connected to a proximal end of the operation arm;
   a lever member attached to the support member so as to rotate with respect to the support member; and
   a finger rest attached to the lever member and on which a finger of an operator is placed.

### Description of Reference Numerals

1: medical manipulator (patient-side apparatus)
2, 302, 502: remote control apparatus (operator-side apparatus)
4: surgical instrument
21a: operation arm
21ba: support member
21bb, 321bb: first lever member
21bc, 321bc: second lever member
21bf, 321bf, 521bf: first switch
21bg, 321bg, 521bg: second switch
21bh, 121bh, 221bh: customized switch
21bj: sensor (angle sensor)
21bl: FPC
21bm: reinforcing plate
21c: flange
22c: camera pedal (foot pedal)
60: manipulator arm (patient-side arm)
100: surgical system (robotic surgical system)
211, 411: first end of the first lever member
212, 412: second end of the first lever member
213: first end of the second lever member
214: second end of the second lever member
215, 415: opposing surface
216, 416: attachment member
216a: first flat plate
216b: second flat plate
321bh: third switch
321bi: switch housing (housing, finger rest)
417: rotary body
418: urging portion (of the roller switch)
419: sensor (of the roller switch)
617: seesaw body
618: urging portion (of the seesaw switch)
619: sensor (of the seesaw switch)
A7: axis (first axis)
A8: axis (second axis, third axis)
A9: axis (third axis, second axis)

## Claims

1. An operator-side apparatus operable to operate a patient-side apparatus including a patient-side arm having a tip end to which a surgical instrument is attached, the operator-side apparatus comprising:
an operation arm;
a support member connected to a proximal end of the operation arm;
a first lever member attached to the support member so as to rotate with respect to the support member; and
a first switch attached to the first lever member to perform a predetermined function.

2. The operator-side apparatus according to claim 1, wherein
a distal end of the support member is connected to the proximal end of the operation arm so as to rotate about a first axis parallel to a longitudinal direction of the support member; and
a proximal end of the first lever member is attached to the support member so as to rotate about a second axis perpendicular to the first axis with respect to the support member.

3. The operator-side apparatus according to claim 2, wherein the first switch is arranged closer to a distal end than the proximal end of the first lever member.

4. The operator-side apparatus according to claim 3, wherein the first switch is arranged at at least one of a first end or a second end of the first lever member in a direction parallel to the second axis.

5. The operator-side apparatus according to claim 1, comprising:
a second lever member facing the first lever member via the support member; and
a second switch attached to the second lever member to perform the predetermined function; wherein
a distal end of the support member is connected to the proximal end of the operation arm so as to rotate about a first axis parallel to a longitudinal direction of the support member; and
a proximal end of the second lever member is attached to the support member so as to rotate about a third axis perpendicular to the first axis with respect to the support member.

6. The operator-side apparatus according to claim 5, wherein the second switch is arranged closer to a distal end than the proximal end of the second lever member.

7. The operator-side apparatus according to claim 5, wherein the second switch is arranged at at least one of a first end or a second end of the second lever member in a direction parallel to the third axis.

8. The operator-side apparatus according to claim 1, wherein the first switch includes a clutch switch to perform a clutch function of preventing an operation of an operator from being transmitted to the patient-side apparatus.

9. The operator-side apparatus according to claim 5, wherein the first switch and the second switch are arranged symmetrically about the first axis of the support member.

10. The operator-side apparatus according to claim 1, wherein the first switch is inclined with respect to an opposing surface of the first lever member facing the support member.

11. The operator-side apparatus according to claim 10, wherein
the first lever member further includes a plate-shaped attachment member attached to the opposing surface to support the first switch such that the first switch is inclined with respect to the opposing surface;
the attachment member includes a first flat plate parallel to the opposing surface, and a second flat plate connected to the first flat plate and inclined so as to be bent with respect to the first flat plate; and
the first switch is attached to the second flat plate.

12. The operator-side apparatus according to claim 1, wherein the first switch is push-button.

13. The operator-side apparatus according to claim 12, wherein the push-button first switch is a clutch switch operable to perform a clutch function of preventing an operation of an operator from being transmitted to the patient-side apparatus, and is a redundant switch.

14. The operator-side apparatus according to claim 1, wherein the first switch is a roller switch.

15. The operator-side apparatus according to claim 14, wherein the roller switch includes a rotary body rotationally operated by an operator, an urging portion to urge the rotary body to an initial position, and a sensor to detect movement of the rotary body.

16. The operator-side apparatus according to claim 1, wherein the first switch is a seesaw switch.

17. The operator-side apparatus according to claim 16, wherein the seesaw switch includes a seesaw body operated by being pressed by a finger of an operator, an urging portion to urge the seesaw body to an initial position, and a sensor to detect movement of the seesaw body.

18. The operator-side apparatus according to claim 1, further comprising:
a customized switch to perform a function other than a clutch function of preventing an operation of an operator from being transmitted to the patient-side apparatus; wherein
a function to be performed by the customized switch is customized by function assignment to the customized switch.

19. The operator-side apparatus according to claim 18, wherein the customized switch is arranged on the first lever member.

20. The operator-side apparatus according to claim 18, wherein the customized switch is arranged on the support member or a flange connecting the proximal end of the operation arm to the support member.

21. The operator-side apparatus according to claim 1, wherein the first switch and a third switch are arranged on the first lever member.

22. The operator-side apparatus according to claim 21, wherein the third switch is inclined with respect to the first switch.

23. The operator-side apparatus according to claim 1, further comprising:
a foot pedal operated by a foot of an operator; wherein
the first switch is operable to perform a function different from the predetermined function by being operated simultaneously with the foot pedal.

24. The operator-side apparatus according to claim 23, wherein the first switch is operable to perform different functions depending on how the first switch is operated when the first switch is operated simultaneously with the foot pedal.

25. The operator-side apparatus according to claim 24, wherein the first switch is operable to perform different functions depending on at least one of an operation time of the first switch or a number of operations of the first switch when the first switch is operated simultaneously with the foot pedal.

26. The operator-side apparatus according to claim 1, wherein the first lever member further includes a plate-shaped attachment member attached to an opposing surface of the first lever member facing the support member to support the first switch on a support surface perpendicular to the opposing surface.

27. The operator-side apparatus according to claim 1, further comprising:
an angle sensor to detect an angle of the first lever member with respect to the support member;
an FPC in which the angle sensor and the first switch are arranged; and
a reinforcing plate arranged at a position of the angle sensor of the FPC.

28. The operator-side apparatus according to claim 1, further comprising:
a housing in which the first switch is arranged; wherein
the housing includes a finger rest on which a finger of an operator is placed.

29. The operator-side apparatus according to claim 28, wherein the housing is arranged on the first lever member operated by an index finger or middle finger of the operator.

30. The operator-side apparatus according to claim 1, wherein the first switch and a second switch operable to perform the predetermined function are arranged on the first lever member.

31. An operator-side apparatus operable to operate a patient-side apparatus including a patient-side arm having a tip end to which a surgical instrument is attached, the operator-side apparatus comprising:
an operation arm;
a support member connected to a proximal end of the operation arm;
a lever member attached to the support member so as to rotate with respect to the support member; and
a finger rest attached to the lever member and on which a finger of an operator is placed.

32. A robotic surgical system comprising:
a patient-side apparatus including a patient-side arm having a tip end to which a surgical instrument is attached; and
an operator-side apparatus operable to operate the patient-side apparatus; wherein
the operator-side apparatus includes:
an operation arm;
a support member connected to a proximal end of the operation arm;
a lever member attached to the support member so as to rotate with respect to the support member; and
a switch attached to the lever member to perform a predetermined function.

33. A robotic surgical system comprising:
a patient-side apparatus including a patient-side arm having a tip end to which a surgical instrument is attached; and
an operator-side apparatus operable to operate the patient-side apparatus; wherein
the operator-side apparatus includes:
an operation arm;
a support member connected to a proximal end of the operation arm;
a lever member attached to the support member so as to rotate with respect to the support member; and
a finger rest attached to the lever member and on which a finger of an operator is placed.
